# EUROPEAN PATENT APPLICATION

(11) **EP 4 410 838 A1**
(43) Date of publication of application: **07.08.2024**
(21) Application number: 22875093.1
(22) Date of filing: 29.09.2022
(51) Int. Cl.: C07K 16/28, A61P 35/00

(54) **ANTI-CD39 ANTIBODY-DRUG CONJUGATE AND USE THEREOF**

(30) Priority: 30.09.2021 CN 202111168038
(71) Applicant: Shanghai Hongcheng Pharmaceutical Co., Ltd., Shanghai 201318 (CN)
(72) Inventor: LI, Zhaohui, Hangzhou, Zhejiang 311112 (CN); WU, Min, Hangzhou, Zhejiang 311112 (CN); ZHANG, Qian, Hangzhou, Zhejiang 311112 (CN); XIE, Zhangming, Hangzhou, Zhejiang 311112 (CN); FANG, Hedi, Hangzhou, Zhejiang 311112 (CN); FANG, Jie, Hangzhou, Zhejiang 311112 (CN); LIU, Wenhui, Hangzhou, Zhejiang 311112 (CN); LIU, Wenhui, Hangzhou, Zhejiang 311112 (CN); LIU, Lixu, Hangzhou, Zhejiang 311112 (CN); LV, Yubin, Hangzhou, Zhejiang 311112 (CN)
(74) Representative: Kador & Partner Part mbB
(86) International application number: PCT/CN2022/122682
(87) International publication number: WO 2023/051712

(57) **Abstract**

The present invention provides an anti-CD39 antibody-drug conjugate or a pharmaceutically acceptable salt or solvate thereof, a pharmaceutical composition comprising the conjugate or the pharmaceutically acceptable salt or solvate thereof, and use thereof as an anti-cancer drug, particularly use thereof in the preparation of a medicament for treating a CD39-mediated disease or disorder. The anti-CD39 antibody-drug conjugate provided by the present application significantly improves the effects and *in-vivolin-vitro* tumor-killing activity of cytotoxin drugs.

## Description

### TECHNICAL FIELD

The present invention relates to an antibody-drug conjugate with a brand-new structure or a pharmaceutically acceptable salt or solvate thereof. In particular, the present invention relates to an anti-CD39 antibody-drug conjugate or a pharmaceutically acceptable salt or solvate thereof, a pharmaceutical composition comprising the antibody-drug conjugate, and use of the antibody-drug conjugate or the pharmaceutical composition in medicines.

### BACKGROUND

Purinergic signal transduction in a tumor microenvironment (TME) plays a key role in immune response regulation. In solid tumors, the death of tumor cells, metabolism, hypoxic stress, and proinflammatory signals all cause a large amount of intracellular adenosine triphosphate (ATP) to be released extracellularly and accumulated, far exceeding the levels in healthy tissues (PloS One. 2008, 3(7): e2599; Curr Opin Pharmacol. 2016, 29: 17-25). Extracellular ATP produces pro-inflammatory stimuli by binding to a P2 purinergic receptor on immune cells so as to play a positive regulatory role in tumor cell killing (Trends Immunol. 2016, 37(7): 427-39). However, tumor cells convert ATP to adenosine by expressing CD39 (also known as ectonucleoside triphosphate diphosphohydrolase 1, NTPDase 1) and CD73 (also known as extracellular-5'-nucleotidase), and then the adenosine inhibits the activity of immune cells by binding to an A2A receptor on immune cells, thereby causing immune escape of tumor cells.

In a tumor microenvironment, CD39 is highly expressed on regulatory T cells (Tregs) and myeloid-derived suppressor cells (MDSCs) and exhibits an inhibitory effect on killer T cells (Blood. 2007, 110(4): 1225-32; Cell MolImmunol. 2017, 14(6): 521-8; Cancer Res. 2016, 76(18): 5241-52; J Immunother Cancer. 2016, 4: 49). The elevated expression of CD39 has also been observed on tumor cells of a variety of hematological tumors and solid tumors, such as thyroid cancer, colorectal cancer, gastric cancer, renal cancer, prostate cancer, testicular cancer, breast cancer, ovarian cancer, melanoma, lymphoma, and the like (Human Protein Atlas). In addition, it has been reported that over-expression of CD39 is associated with poor cancer prognosis (Int J Clin Exp Pathol. 2015, 8(11): 14757-14764). Thus, CD39 is considered a very potential therapeutic target. At present, a plurality of monoclonal antibody drugs against CD39 targets have entered the clinical research stage, such as monoclonal antibodies IPH5201 and TTX-030, but there is still a clinical need for developing drug molecules with better efficacy.

An antibody-drug conjugate (ADC) is a targeted therapy in which a monoclonal antibody or an antibody fragment binds to a toxin with cytotoxicity through a stable linker, so that the toxin is efficiently delivered to a tumor site by sufficiently utilizing the targeted binding of the antibody to a tumor cell surface antigen. The ADC drug consists of three moieties: an antibody, a linker, and a toxin, wherein the antibody moiety determines the specificity of the ADC drug, which includes not only specific target binding, but also efficient endocytosis. The type and chemical properties of the linker determine the conjugation quantity of the toxin, the *in-vivo* release mechanism, and pharmacokinetics of the ADC drug, and also greatly affect a therapeutic index (an efficacy/toxicity ratio). The type of the toxin determines the mechanism of action of the ADC drug killing tumor cells, such as targeting tubulins, inhibiting microtubule dynamics, or targeting DNA grooves, and disrupting DNA double helix. The toxicity and chemical characteristics of the toxin itself also affect the toxin conjugation quantity and stability of the ADC drug to a certain extent, and different types of tumor cells often exhibit different sensitivities to different toxins, which all affect the therapeutic index of the ADC drug. Thus, the efficacy and safety of ADC obtained by combination of a new antibody with different linkers and toxins are uncertain and difficult to predict.

At present, a variety of ADC drugs have been approved on the market. For example, Kadcyla and Enhertu are ADC drugs formed by respectively conjugating an HER2-targeting monoclonal antibody with DM1 and Deruxtecan. 1-394 (named F1 in the present application) is an anti-CD39 monoclonal antibody developed by Innate Pharma, which has a related patent publication number WO2019096900A1, and is in phase 1 clinical trial at present. At present, no ADC drug targeting CD39 has been disclosed that is under clinical study. Those skilled in the art are committed to developing new and effective CD39-targeting ADC drugs.

### SUMMARY

The objective of the present invention is to provide an antibody-drug conjugate represented by general formula (I) or a pharmaceutically acceptable salt or solvate thereof: 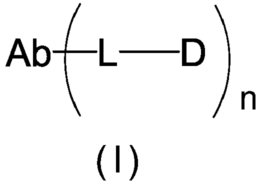 wherein
Ab is an anti-CD39 antibody or an antigen-binding fragment thereof;
L is a linker;
D is a cytotoxin molecule;
"-" is a bond;
n is an average conjugation quantity of the cytotoxin molecule conjugated to the antibody, and is also an average value of the ratio of the cytotoxin molecule to the antibody (drug antibody ratio), also called a DAR value, wherein 0 < n ≤ 10, preferably 0.8 ≤ n ≤ 5.5, and n can be an integer or a non-integer.

In a preferred embodiment of the present invention, provided is an antibody-drug conjugate represented by general formula (I) or a pharmaceutically acceptable salt or solvate thereof, wherein the anti-CD39 antibody or the antigen-binding fragment thereof comprises the following CDRs:
an HCDR1, the amino acid sequence of which is set forth in SEQ ID NO: 1, or which comprises the amino acid sequence set forth in SEQ ID NO: 1;
an HCDR2, the amino acid sequence of which is set forth in SEQ ID NO: 2, or which comprises the amino acid sequence set forth in SEQ ID NO: 2;
an HCDR3, the amino acid sequence of which is set forth in SEQ ID NO: 3, or which comprises the amino acid sequence set forth in SEQ ID NO: 3;
an LCDR1, the amino acid sequence of which is set forth in SEQ ID NO: 4, or which comprises the amino acid sequence set forth in SEQ ID NO: 4;
an LCDR2, the amino acid sequence of which is set forth in SEQ ID NO: 5, or which comprises the amino acid sequence set forth in SEQ ID NO: 5; and
an LCDR3, the amino acid sequence of which is set forth in SEQ ID NO: 6, or which comprises the amino acid sequence set forth in SEQ ID NO: 6.

In a preferred embodiment of the present invention, provided is an antibody-drug conjugate represented by general formula (I) or a pharmaceutically acceptable salt or solvate thereof, wherein the amino acid sequence of a heavy chain variable region of the anti-CD39 antibody or the antigen-binding fragment thereof is set forth in SEQ ID NO: 7, or has at least 90%, at least 95%, at least 96%, at least 97%, at least 98% or at least 99% sequence identity to SEQ ID NO: 7; and/or the amino acid sequence of a light chain variable region of the anti-CD39 antibody or the antigen-binding fragment thereof is set forth in SEQ ID NO: 8, or has at least 90%, at least 95%, at least 96%, at least 97%, at least 98% or at least 99% sequence identity to SEQ ID NO: 8.

In a preferred embodiment of the present invention, provided is an antibody-drug conjugate represented by general formula (I) or a pharmaceutically acceptable salt or solvate thereof, wherein the anti-CD39 antibody or the antigen-binding fragment thereof is a murine antibody or a fragment thereof, further comprising a heavy chain constant region of a murine IgG1, IgG2, IgG3 or IgG4 or a variant thereof; and/or further comprising a light chain constant region of a murine κ or λ chain or a variant thereof.

In a preferred embodiment of the present invention, provided is an antibody-drug conjugate represented by general formula (I) or a pharmaceutically acceptable salt or solvate thereof, wherein the anti-CD39 antibody or the antigen-binding fragment thereof is a chimeric antibody or an antigen-binding fragment thereof, further comprising a heavy chain constant region of a human IgG1, IgG2, IgG3 or IgG4 or a variant thereof, preferably comprising a human IgG4 heavy chain constant region; and/or further comprising a light chain constant region of a human κ or λ chain or a variant thereof, preferably a human κ light chain constant region.

In a preferred embodiment of the present invention, provided is an antibody-drug conjugate represented by general formula (I) or a pharmaceutically acceptable salt or solvate thereof, wherein the amino acid sequence of a heavy chain of the chimeric antibody or the antigen-binding fragment thereof is set forth in SEQ ID NO: 9, or has at least 90%, at least 95%, at least 96%, at least 97%, at least 98% or at least 99% sequence identity to SEQ ID NO: 9; and/or the amino acid sequence of a light chain of the antibody is set forth in SEQ ID NO: 10, or has at least 90%, at least 95%, at least 96%, at least 97%, at least 98% or at least 99% sequence identity to SEQ ID NO: 10.

In a preferred embodiment of the present invention, provided is an antibody-drug conjugate represented by general formula (I) or a pharmaceutically acceptable salt or solvate thereof, wherein the anti-CD39 antibody or the antigen-binding fragment thereof is a humanized antibody or an antigen-binding fragment thereof, further comprising a heavy chain FR of a human IgG1, IgG2, IgG3 or IgG4 or a variant thereof, preferably comprising an FR of a human germline heavy chain IGHV1-2*02 or a variant thereof; and/or further comprising a light chain FR of a human κ or λ chain or a variant thereof, preferably an FR of a human germline light chain IGKV1-33*01 or a variant thereof.

In a preferred embodiment of the present invention, provided is an antibody-drug conjugate represented by general formula (I) or a pharmaceutically acceptable salt or solvate thereof, wherein the amino acid sequence of a heavy chain variable region of the humanized antibody or the antigen-binding fragment thereof is set forth in SEQ ID NO: 11, 12, 13, 14 or 15, or has at least 90%, at least 95%, at least 96%, at least 97%, at least 98% or at least 99% sequence identity to SEQ ID NO: 11, 12, 13, 14 or 15; and/or the amino acid sequence of a light chain variable region of the humanized antibody or the antigen-binding fragment thereof is set forth in SEQ ID NO: 16 or 17, or has at least 90%, at least 95%, at least 96%, at least 97%, at least 98% or at least 99% sequence identity to SEQ ID NO: 16 or 17.

In a preferred embodiment of the present invention, provided is an antibody-drug conjugate represented by general formula (I) or a pharmaceutically acceptable salt or solvate thereof, wherein the amino acid sequence of the heavy chain variable region of the humanized antibody or the antigen-binding fragment thereof is set forth in SEQ ID NO: 11, or has at least 90%, at least 95%, at least 96%, at least 97%, at least 98% or at least 99% sequence identity to SEQ ID NO: 11, and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 12, or has at least 90%, at least 95%, at least 96%, at least 97%, at least 98% or at least 99% sequence identity to SEQ ID NO: 12, and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 13, or has at least 90%, at least 95%, at least 96%, at least 97%, at least 98% or at least 99% sequence identity to SEQ ID NO: 13, and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 14, or has at least 90%, at least 95%, at least 96%, at least 97%, at least 98% or at least 99% sequence identity to SEQ ID NO: 14, and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 15, or has at least 90%, at least 95%, at least 96%, at least 97%, at least 98% or at least 99% sequence identity to SEQ ID NO: 15, and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 11, or has at least 90%, at least 95%, at least 96%, at least 97%, at least 98% or at least 99% sequence identity to SEQ ID NO: 11, and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17; or
the amino acid sequence of the heavy chain variable region of the humanized antibody or the antigen-binding fragment thereof is set forth in SEQ ID NO: 12, or has at least 90%, at least 95%, at least 96%, at least 97%, at least 98% or at least 99% sequence identity to SEQ ID NO: 12, and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17; or
the amino acid sequence of the heavy chain variable region of the humanized antibody or the antigen-binding fragment thereof is set forth in SEQ ID NO: 13, or has at least 90%, at least 95%, at least 96%, at least 97%, at least 98% or at least 99% sequence identity to SEQ ID NO: 13, and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17; or
the amino acid sequence of the heavy chain variable region of the humanized antibody or the antigen-binding fragment thereof is set forth in SEQ ID NO: 14, or has at least 90%, at least 95%, at least 96%, at least 97%, at least 98% or at least 99% sequence identity to SEQ ID NO: 14, and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17; or
the amino acid sequence of the heavy chain variable region of the humanized antibody or the antigen-binding fragment thereof is set forth in SEQ ID NO: 15, or has at least 90%, at least 95%, at least 96%, at least 97%, at least 98% or at least 99% sequence identity to SEQ ID NO: 15, and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17.

In a preferred embodiment of the present invention, provided is an antibody-drug conjugate represented by general formula (I) or a pharmaceutically acceptable salt or solvate thereof, wherein the humanized antibody or the antigen-binding fragment thereof further comprises a heavy chain constant region of the human IgG1, IgG2, IgG3 or IgG4 or the variant thereof, preferably comprises a human IgG4 heavy chain constant region, wherein more preferably, the amino acid sequence of the human IgG4 heavy chain constant region is set forth SEQ ID NO: 18, or has at least 90%, at least 95%, at least 96%, at least 97%, at least 98% or at least 99% sequence identity to SEQ ID NO: 18.

In a preferred embodiment of the present invention, provided is an antibody-drug conjugate represented by general formula (I) or a pharmaceutically acceptable salt or solvate thereof, wherein the humanized antibody or the antigen-binding fragment thereof further comprises a light chain constant region of the human κ or λ chain or the variant thereof, preferably a human κ light chain constant region, wherein more preferably, the amino acid sequence of the human κ light chain constant region is set forth in SEQ ID NO: 19, or has at least 90%, at least 95%, at least 96%, at least 97%, at least 98% or at least 99% sequence identity to SEQ ID NO: 19.

In a preferred embodiment of the present invention, provided is an antibody-drug conjugate represented by general formula (I) or a pharmaceutically acceptable salt or solvate thereof, wherein the linker L is represented by general formula (L): 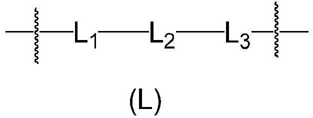 wherein
L₁ is selected from 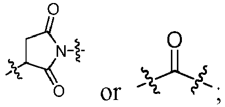
L₂ is 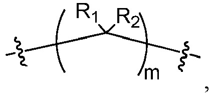 wherein R₁ and R₂ are each independently selected from H, alkyl, haloalkyl, or halogen, and m is 1-8, preferably 2-5; L₂ is preferably 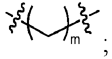 or L₂ is 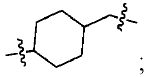
L₃ is selected from

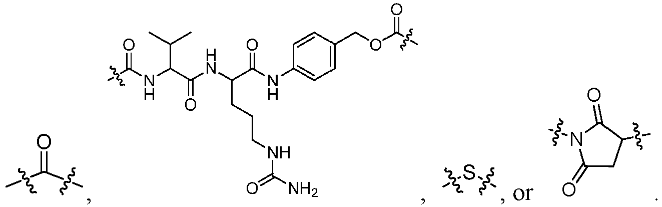

In a preferred embodiment of the present invention, provided is an antibody-drug conjugate represented by general formula (I) or a pharmaceutically acceptable salt or solvate thereof, wherein the linker L is selected from the following structures: 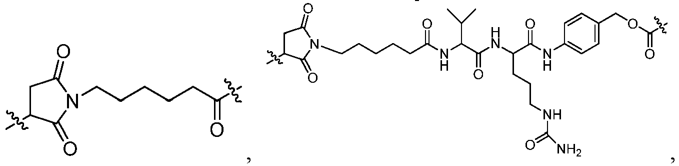 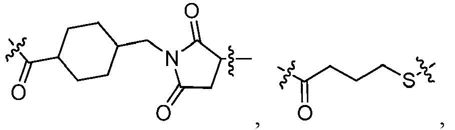 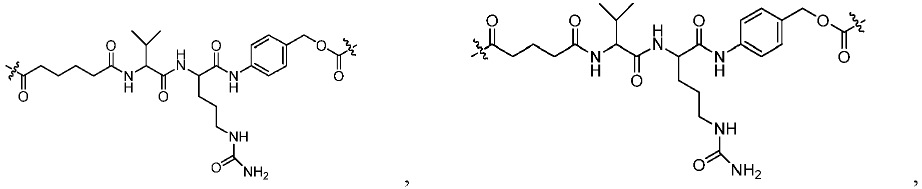 and

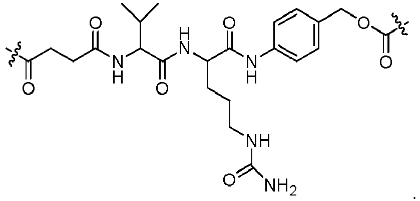

In a preferred embodiment of the present invention, provided is an antibody-drug conjugate represented by general formula (I) or a pharmaceutically acceptable salt or solvate thereof, wherein the cytotoxin molecule D is selected from a chemotherapeutic agent, a DNA alkylating agent, a tubulin inhibitor, a topoisomerase inhibitor, an antibiotic, or a cytotoxic agent of a radioisotope.

In a preferred embodiment of the present invention, provided is an antibody-drug conjugate represented by general formula (I) or a pharmaceutically acceptable salt or solvate thereof, wherein the cytotoxin molecule D is selected from the following structures: 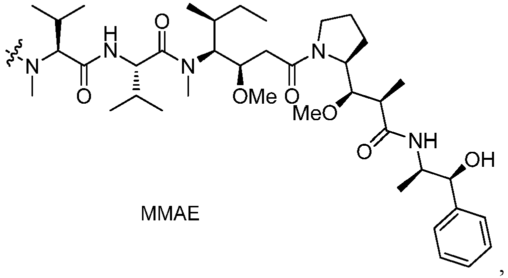 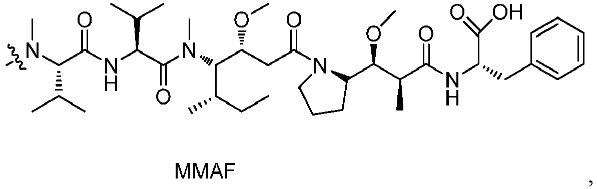 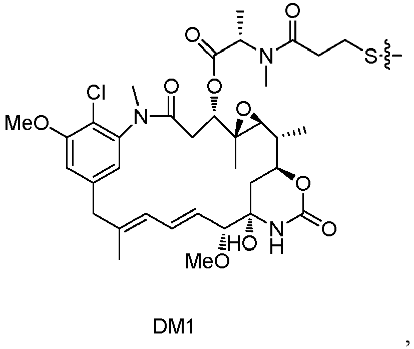 and

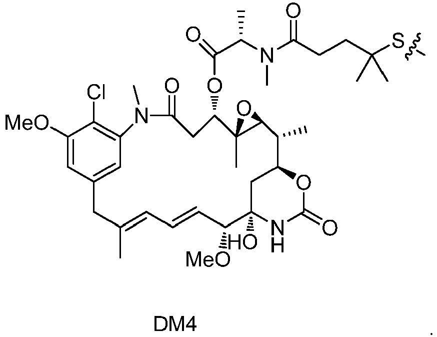

In a preferred embodiment of the present invention, provided is an antibody-drug conjugate represented by general formula (I) or a pharmaceutically acceptable salt or solvate thereof, including an antibody-drug conjugate represented by general formula (II) or a pharmaceutically acceptable salt or solvate thereof: 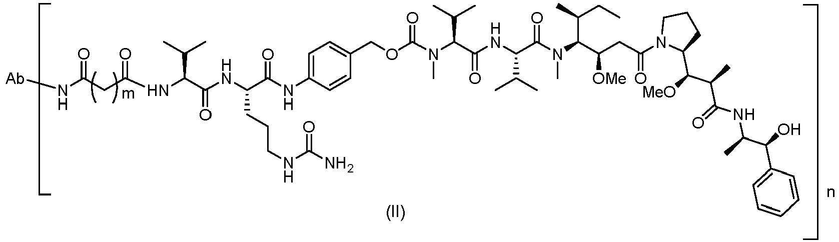 wherein m is 1-8, preferably 2-4, 0 < n ≤ 10, preferably 0.8 ≤ n ≤ 3, Ab is as defined in general formula (I) according to any one of the embodiments described above, and the linker is linked to a lysine side chain of Ab.

It can be seen from the common knowledge in the art that among 20 common amino acids, the amino acid side chain having reactivity for condensation reaction with the linker and forming an imino residue after the reaction is only the lysine side chain. Therefore, in combination with the structure of general formula (II) and the common knowledge in the art, it can be known that the linker in general formula (II) is linked to a lysine side chain of Ab, and meanwhile, the lysine side chain of the antibody reacts with the linker to form an imino group, the quantity of which is the same as that of cytotoxin molecules. Therefore, the imino group is recorded at the very left inside the square brackets of general formula (II).

In a preferred embodiment of the present invention, provided is an antibody-drug conjugate represented by general formula (I) or a pharmaceutically acceptable salt or solvate thereof, including an antibody-drug conjugate represented by general formula (III) or a pharmaceutically acceptable salt or solvate thereof: 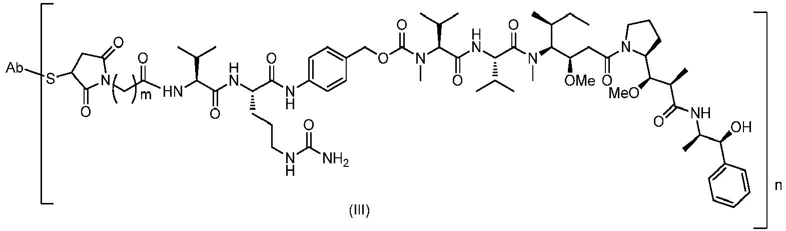 wherein m is 1-8, preferably 3-5; 0 < n ≤ 8, Ab is as defined in general formula (I) according to any one of the embodiments described above, and the linker is linked to a cysteine side chain of Ab.

It can be seen from the common knowledge in the art that among 20 common amino acids, the amino acid side chain having reactivity for condensation reaction with the linker and forming a -S-residue after the reaction is only the cysteine side chain. Therefore, in combination with the structure of general formula (II) and the common knowledge in the art, it can be known that the linker in general formula (II) is linked to the cysteine side chain of Ab, and meanwhile, the cysteine side chain of the antibody reacts with the linker to form a -S- residue, the quantity of which is the same as that of cytotoxin molecules. Therefore, the -S- residue is recorded at the very left inside the square brackets of general formula (II).

In a preferred embodiment of the present invention, the antibody-drug conjugate or the pharmaceutically acceptable salt or solvate thereof described herein includes, but is not limited to: 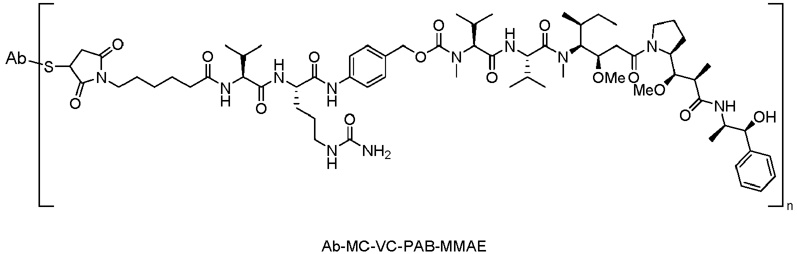 wherein 0 < n ≤ 8, Ab is as defined in general formula (I) according to any one of the embodiments described above, and the linker is linked to a cysteine side chain of Ab; or 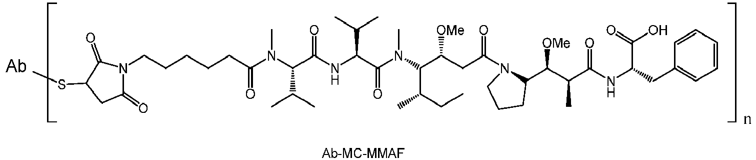 wherein 0 < n ≤ 8, Ab is as defined in general formula (I) according to any one of the embodiments described above, and the linker is linked to a cysteine side chain of Ab; or 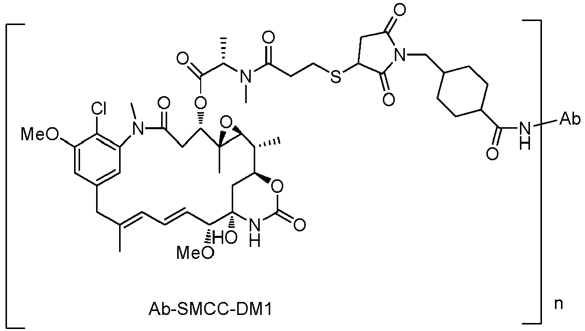 wherein 0 < n ≤ 10, Ab is as defined in general formula (I) according to any one of the embodiments described above, and the linker is linked to a lysine side chain of Ab; or 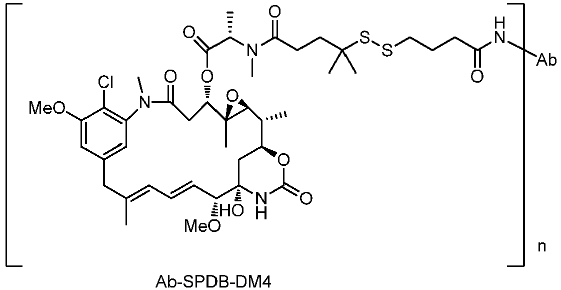 wherein 0 < n ≤ 10, Ab is as defined in general formula (I) according to any one of the embodiments described above, and the linker is linked to a lysine side chain of Ab; or 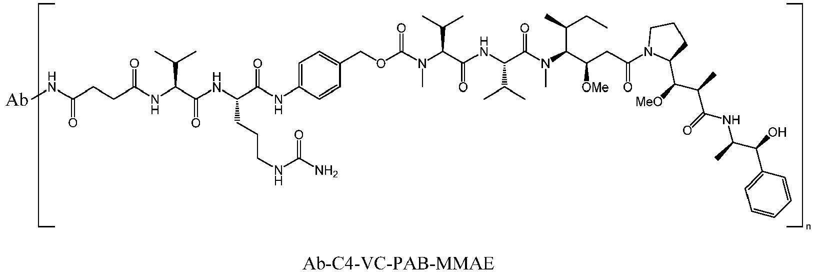 wherein 0 < n ≤ 10, preferably 0.8 ≤ n ≤ 3, Ab is as defined in general formula (I) according to any one of the embodiments described above, and the linker is linked to a lysine side chain of Ab; or 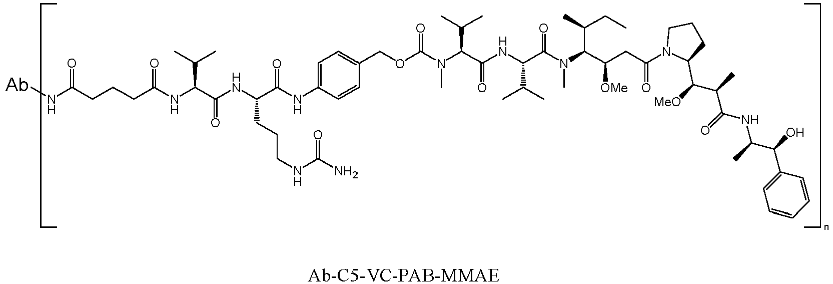 wherein 0 < n ≤ 10, preferably 0.8 ≤ n ≤ 3, Ab is as defined in general formula (I) according to any one of the embodiments described above, and the linker is linked to a lysine side chain of Ab; or 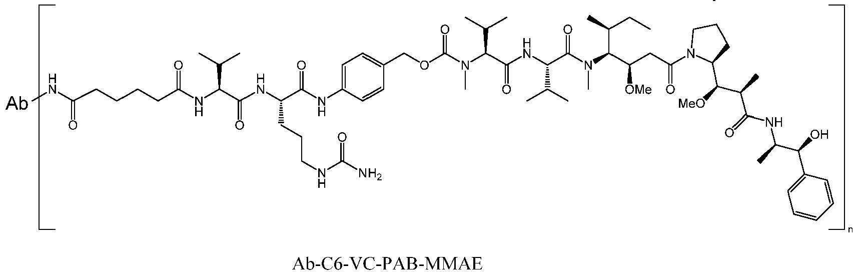 wherein 0 < n ≤ 10, preferably 0.8 ≤ n ≤ 3, Ab is as defined in general formula (I) according to any one of the embodiments described above, and the linker is linked to a lysine side chain of Ab.

In a more preferred embodiment, the antibody-drug conjugate described herein is:
(1) F314-MC-VC-PAB-MMAE with a structure shown in the following formula: 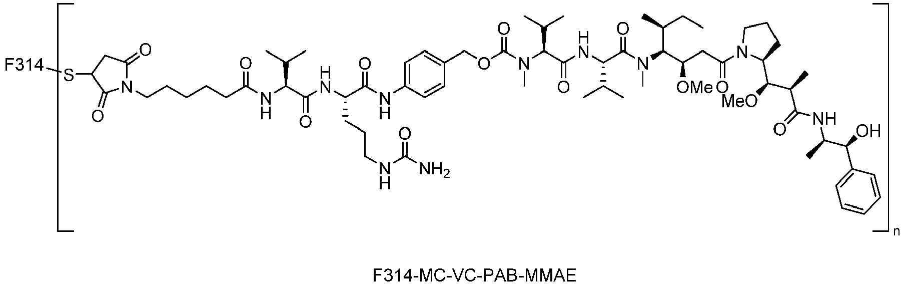 wherein n is 3.8-4.2, preferably 3.9-4.1, more preferably 4.0, and the linker is linked to a cysteine side chain of Ab;
(2) F314-MC-MMAF with a structure shown in the following formula: 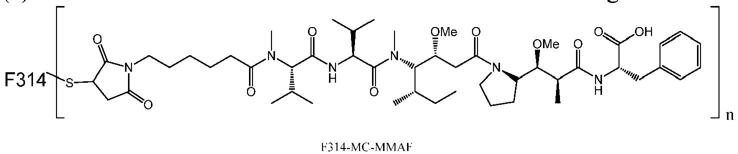 wherein n is 1.3-1.7, preferably 1.4-1.6, more preferably 1.5, and the linker is linked to a cysteine side chain of Ab;
(3) F314-SMCC-DM1 with a structure shown in the following formula: 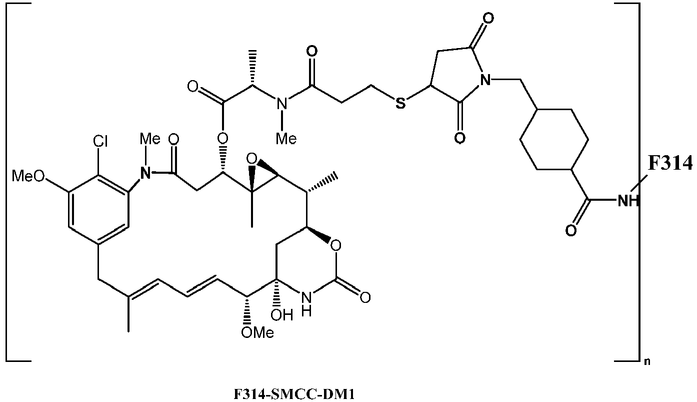 wherein n is 4.8-5.2, preferably 4.9-5.1, more preferably 5.0, and the linker is linked to a lysine side chain of Ab;
(4) F314-SPDB-DM4 with a structure shown in the following formula: 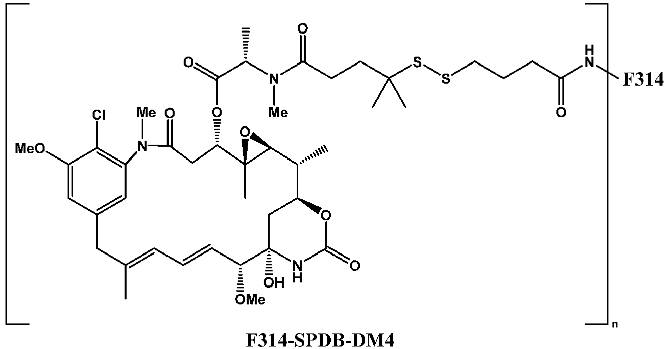 wherein n is 3.6-4.0, preferably 3.7-3.9, more preferably 3.8, and the linker is linked to a lysine side chain of Ab;
(5) F314-C4-VC-PAB-MMAE with a structure shown in the following formula: 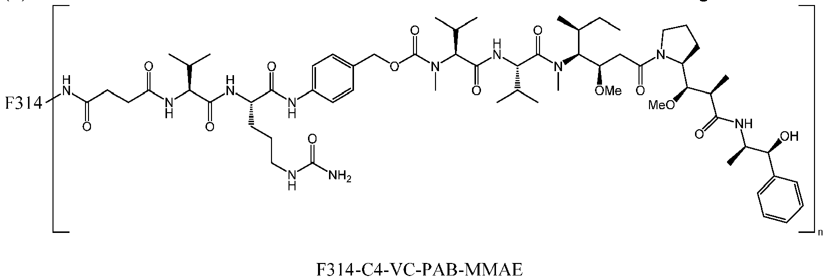 wherein n is 1.9-2.3, preferably 2.0-2.2, more preferably 2.1, and the linker is linked to a lysine side chain of Ab;
(6) F314-C5-VC-PAB-MMAE with a structure shown in the following formula: 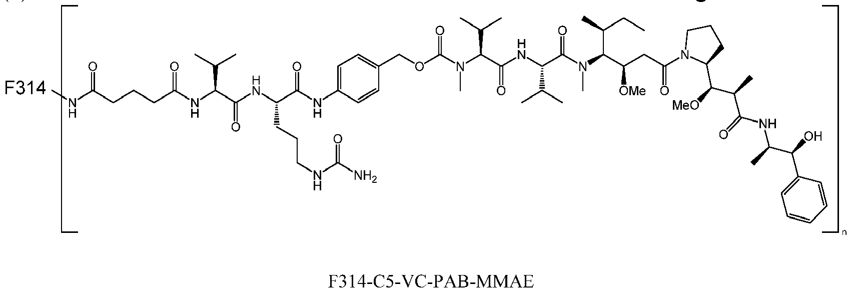 wherein n is 0.5-3.5, preferably 0.8-3.0, more preferably 0.8, 1.8, 2.3, or 3.0, and the linker is linked to a lysine side chain of Ab; or
(7) F314-C6-VC-PAB-MMAE with a structure shown in the following formula:

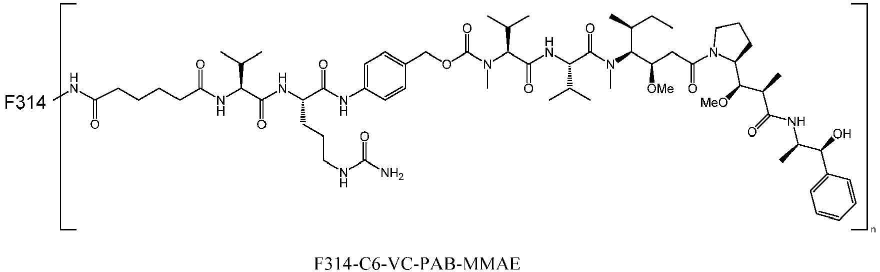

wherein n is 1.9-2.3, preferably 2.0-2.2, more preferably 2.1, and the linker is linked to a lysine side chain of Ab;
wherein, in each formula, F314 represents a humanized monoclonal antibody F314, which has a heavy chain variable region with the amino acid sequence set forth in SEQ ID NO: 14, a light chain variable region with the amino acid sequence set forth in SEQ ID NO: 17, a heavy chain constant region with the amino acid sequence set forth in SEQ ID NO: 18, and a light chain constant region with the amino acid sequence set forth in SEQ ID NO: 19.

The present invention further provides a pharmaceutical composition, comprising the antibody-drug conjugate or the pharmaceutically acceptable salt or solvate thereof as described above, and a pharmaceutically acceptable excipient, diluent or carrier.

The present invention further provides use of the antibody-drug conjugate or the pharmaceutically acceptable salt or solvate thereof or the pharmaceutical composition comprising the same as described above in the preparation of a medicament for treating a CD39-mediated disease or disorder, wherein the disease or disorder is preferably a cancer, preferably lymphoma (such as follicular lymphoma and mantle cell lymphoma), multiple myeloma, thyroid cancer, colorectal cancer, gastric cancer, renal cancer, prostate cancer, testicular cancer, breast cancer, ovarian cancer, or melanoma.

The present invention further provides a method for treating and preventing a CD39-mediated disease or disorder, comprising administering to a patient in need thereof a therapeutically effective amount of the antibody-drug conjugate or the pharmaceutically acceptable salt or solvate thereof or the pharmaceutical composition comprising the same as described above, wherein the disease or disorder is preferably a cancer, preferably lymphoma (such as follicular lymphoma and mantle cell lymphoma), multiple myeloma, thyroid cancer, colorectal cancer, gastric cancer, renal cancer, prostate cancer, testicular cancer, breast cancer, ovarian cancer, or melanoma.

The anti-CD39 antibody-drug conjugate designed in the present invention not only retains the affinity and endocytosis activity of the monoclonal antibody but also achieves a direct targeted killing effect on tumor cells through the conjugated cytotoxin molecule, such that the activity of ADC is significantly increased compared with that of the monoclonal antibody.

### BRIEF DESCRIPTION OF THE DRAWINGS

FIG. 1 shows the *in-vitro* killing activity of naked antibodies and different ADCs against CD39-positive tumor cells.
FIG. 2 shows the *in-vivo* anti-tumor activity of a naked antibody and different ADCs in mice inoculated with CD39-positive tumor cells.
FIG. 3 shows the *in-vitro* killing activity of a naked antibody and different ADCs against CD39-negative tumor cells.
FIG. 4 shows the affinity activity of a naked antibody and different ADCs for CD39-positive tumor cells.
FIG. 5 shows the endocytosis activity of a naked antibody and different ADCs.
FIG. 6 shows the *in-vitro* killing activity of F314-C5-VC-PAB-MMAE with different DAR values against CD39-positive tumor cells.

### DETAILED DESCRIPTION

### I. Terminology

In order to facilitate the understanding of the present invention, certain technical and scientific terms are specifically defined below. Unless otherwise specifically defined herein, all other technical and scientific terms used herein have the meanings generally understood by those of ordinary skill in the art to which the present invention belongs.

"CD39" and "CD39 antigen" as well as "CD39 protein" can be used interchangeably in the present invention. CD39 is also called ectonucleoside triphosphate diphosphohydrolase 1 (gene: ENTPD1; protein: NTPDase1, see www.ncbi.nlm.nih.gov/gene/953). CD39 was also referred to as ATPDase and SPG64. These terms described above may be used interchangeably. Unless otherwise indicated, the terms include any variants, subtypes and species homologs of human CD39 that are expressed naturally by cells or by cells transfected with the CD39 gene. In the present invention, the CD39 protein has a UniProtKB/Swiss-Prot accession number of P49961.1.

The "antibody-drug conjugate" (ADC) of the present invention means that a monoclonal antibody or an antibody fragment thereof is linked to a biologically active cytotoxin by a stable chemical linker compound.

The term "pharmaceutically acceptable salt" refers to a salt of the antibody-cytotoxic drug conjugate of the present invention, which has safety and effectiveness when being used in the body of a mammal and possesses requisite biological activity. The antibody-drug conjugate of the present invention at least contains one amino group and therefore can form a salt with an acid.

The term "solvate" refers to a pharmaceutically acceptable solvate formed by the antibody-drug conjugate of the present invention with one or more solvent molecules.

The single-letter codes for amino acids used herein are as described in J. biol. chem, 243, p3558 (1968).

The antibody described herein refers to an immunoglobulin which is in a tetrapeptide chain structure formed by connection of two identical heavy chains and two identical light chains via interchain disulfide bonds. The heavy chain constant regions of an immunoglobulin differ in their amino acid composition and arrangement, and therefore have different antigenicities. Accordingly, the immunoglobulin can be divided into five classes, otherwise called isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain.

In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions include 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs which are arranged in sequence from the amino-terminus to the carboxyl-terminus: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3. The CDR amino acid residues of the LCVRs and HCVRs of the antibody or the antigen-binding fragment thereof described herein accord with the known Kabat numbering scheme (such as LCDR1-3 and HCDR1-3), or accord with the Chothia numbering scheme, in terms of quantity and position.

The monoclonal antibody or mAb described herein refers to an antibody obtained from a single clonal cell strain which is not limited to eukaryotic, prokaryotic, or phage clonal cell strains. The monoclonal antibody or the antigen-binding fragment thereof can be obtained by, for example, a hybridoma technology, a recombination technology, a phage display technology, a synthetic technology (such as CDR-grafting), or recombination using other existing technologies.

The term "murine antibody" in the present invention is a monoclonal antibody against human CD39 prepared by using mice according to the knowledge and skill in the art. In a preferred embodiment of the present invention, the murine CD39 antibody or the antigen-binding fragment thereof may further comprise a light chain constant region of a murine κ or λ chain or a variant thereof, or further comprise a heavy chain constant region of a murine IgG1, IgG2, IgG3 or IgG4 or a variant thereof.

The term "chimeric antibody" refers to an antibody obtained by fusing a variable region of a murine antibody with a constant region of a human antibody, which can alleviate an immune response induced by the murine antibody. To establish the chimeric antibody, a hybridoma secreting a murine specific monoclonal antibody is first established, and then variable region genes are cloned from mouse hybridoma cells and then cloned into the constant region genes of the human antibody for recombinant expression.

The term "humanized antibody", also known as CDR-grafted antibody humanization, refers to an antibody generated by grafting murine CDR sequences into a variable region framework of a human antibody, i.e., a different type of human germline antibody framework sequence. The humanized antibody can overcome strong immune response induced by chimeric antibodies due to their carrying a large amount of mouse protein components. Such framework sequences can be obtained from public DNA databases or published references that include germline antibody gene sequences. For example, germline DNA sequences of genes of human heavy and light chain variable regions can be found in the "VBase" human germline sequence database (available at the Internet address www.mrccpe.com.ac.uk/vbase), as well as in Kabat, E. A. et al., 1991 Sequences of Proteins of Immunological Interest, 5th Edition. To avoid the decrease in activity caused by the decrease in immunogenicity, the variable regions of the human antibody can be subjected to a minimum reverse mutation to maintain the activity.

"Antigen-binding fragment" described herein refers to a fragment with antigen-binding activity, including a Fab fragment, a Fab' fragment and a F(ab')₂ fragment, as well as an Fv fragment and an scFv fragment that bind to human CD39; the antigen-binding fragment comprises one or more CDRs of the antibody of the present invention selected from SEQ ID NO: 1 to SEQ ID NO: 6. The Fv fragment comprises the heavy and light chain variable regions of the antibody but lacks constant regions, and is the smallest antibody fragment having all antigen-binding sites. Generally, the Fv antibody also comprises a polypeptide linker between VH and VL domains, and is capable of forming a structure required for antigen binding. Different linkers can also be used to link two antibody variable regions into a polypeptide chain, known as a single chain antibody or single chain Fv (scFv).

The antibody or the antigen-binding fragment of the present invention can be prepared and purified by conventional methods. For example, cDNA sequences encoding amino acid sequences of heavy and light chains can be cloned and recombined into an expression vector pcDNA3.4. The recombinant immunoglobulin expression vector can be stably transfected into CHO cells. Positive clones are subjected to amplification culture in a serum-free medium of a bioreactor to produce antibodies. The culture medium with the secreted antibodies can be purified by conventional techniques.

The term "sequence identity" refers to the sequence similarity between two polynucleotide sequences or between two polypeptides. When positions in two compared sequences are all occupied by the same base or amino acid monomer subunit, for example, if the position of each of two DNA molecules is occupied by adenine, the molecules are identical at that position. The identity percentage between two sequences is a function of the number of matched or homologous positions shared by the two sequences divided by the number of the compared positions × 100. For example, if 9 out of 10 positions are matched or homologous when two sequences are optimally aligned, the two sequences have 90% identity. In general, the comparison is made when two aligned sequences give the greatest identity percentage. Those skilled in the art can determine the quantity of base changes or amino acid changes as indicated by sequence identity percentage.

The term "conservative modification" or "conservative replacement or substitution" refers to replacement of amino acids in a protein with other amino acids having similar characteristics (such as charge, the size of side chain, hydrophobicity/hydrophilicity, backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with a similar structure or function is unlikely to disrupt the biological activity. In the present invention, a variant of the light or heavy chain of the antibody means that a "conservative modification" or "conservative replacement or substitution" of 0-10 amino acids is occurred in the light or heavy chain, and those skilled in the art can expect that the variant has substantially the same activity as that before the modification or substitution. Furthermore, the variant of the light or heavy chain of the antibody in the present invention also comprises a result after a back mutation, i.e., a back mutation of amino acids of individual human templates of the humanized antibody FR into murine amino acids at corresponding sites. Those skilled in the art can expect that the variant has similar, comparable or better activity compared with the humanized antibody prior to the back mutation and a murine antibody comprising the same CDRs.

"Affinity" or "binding" refers to the summed strength of non-covalent interactions between a single binding site of a molecule (e.g., an antibody) and its binding partner (e.g., an antigen). Unless otherwise indicated, "binding affinity" used herein refers to intrinsic binding affinity that reflects a 1:1 interaction between members (e.g., an antibody and an antigen) of a binding pair. The affinity of a molecule X for its binding partner Y is usually expressed as a dissociation constant (KD). Affinity can be determined by conventional methods known in the art, including those described herein, for example, using a surface plasmon resonance (SPR) technology or other instruments.

"Specific binding or specifically binds to", "specific for", "selectively binds", and "selective for" a particular antigen (e.g., a polypeptide target) or an epitope of a particular antigen means binding that is distinct in measurement from non-specific or non-selective interaction. Specific binding can be determined, for example, by measuring the binding of the molecule as compared with the binding of a control molecule. Specific binding can also be determined by competition with a control molecule (such as an excess of unlabeled target) that is similar to the target. The term "kd" (sec⁻¹) used herein refers to a dissociation rate constant for a particular antibody-antigen interaction. This value is also referred to as a k dissociation value. The term "ka" (M⁻¹ × sec⁻¹) used herein refers to an association rate constant for a particular antibody-antigen interaction. This value is also referred to as a k association value. The term "KD" (M) used herein refers to a dissociation equilibrium constant for a particular antibody-antigen interaction. KD = kd / ka.

"Effective amount" includes an amount sufficient to ameliorate or prevent a symptom or sign of a medical disorder. The effective amount also refers to an amount sufficient to allow or facilitate diagnosis. The effective amount for a particular patient or veterinary subject may vary depending on factors such as the disorder to be treated, the general health of the patient, the method and route and dose of administration, and the severity of side effects. The effective amount may be the maximum dose or administration regimen to avoid significant side effects or toxic effects.

The term "carrier" used for the drug described herein refers to a system that can alter the manner of the drug entering a human body and the distribution of the drug in the human body, control the release rate of the drug, and deliver the drug to a targeted organ. The drug carrier release and targeted system can reduce drug degradation and loss, reduce side effects, and improve bioavailability.

"Cytotoxin molecule" refers to any substance capable of creating a deleterious effect on the growth or proliferation of cells, such as the small-molecule drug groups MMAE, MMAF, DM1 and DM4 in the present invention.

"Naked antibody" refers to an antibody or an antigen-binding fragment thereof that is not linked to any cytotoxin molecule.

"Chemotherapeutic agent" refers to a chemical compound useful in the treatment of cancers. The definition also includes anti-hormonal agents that act to modulate, reduce, block or inhibit the effects of hormones that can promote cancer growth, and are often in the form of systematic or systemic treatment. They may themselves be hormones.

The term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 2,2-diethylhexyl and various branched isomers thereof, and the like.

The term "bond" refers to a covalent bond represented by "-". 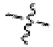 means linking to other groups via covalent bonds; for example, in the present invention, it means covalent linking between the antibody and the linker and between the linker and the cytotoxin molecule.

"Linker" refers to a chemical module comprising a covalent bond or an atom chain that covalently attaches an antibody to a drug module, and is an "extender unit" that links an antibody to another linker member or drug module. In some embodiments, the linker unit may be an amino acid unit. In one such embodiment, the amino acid unit allows for cleavage of the linker by a protease, thereby facilitating the release of the drug from the antibody-drug conjugate or the pharmaceutically acceptable salt or solvate thereof upon exposure to an intracellular protease (such as a lysosomal enzyme).

"Loading of ADC" (drug/antibody ratio (DAR)) can be controlled in different ways, for example, by: (i) limiting the molar amount of a drug-linker intermediate or a linker reagent relative to an antibody, (ii) limiting the time or temperature of a conjugation reaction, (iii) cysteine thiol-modified moieties or limiting reduction conditions, (iv) performing engineering modification on the amino acid sequence of the antibody by a recombinant technology, so that the number and position of cysteine residues are altered in order to control the number and/or position of linker-drug attachments.

Provided are certain methods for preparing antibody-cytotoxic drug conjugates or pharmaceutically acceptable salts or solvates thereof. The ADC of general formula I can be prepared by several routes using organic chemical reactions, conditions and reagents known to those skilled in the art, including: (1) reacting a nucleophilic group of an antibody with a bivalent linker reagent via a covalent bond to form Ab-L, and then reacting Ab-L with a drug module D; and (2) reacting the nucleophilic group of the drug module with the bivalent linker reagent via a covalent bond to form D-L, and then reacting D-L with the nucleophilic group of the antibody.

SKOV-3 is a human ovarian cancer cell line which does not express human CD39 on its cell surface, and is used for detecting the *in-vitro* killing activity effect of anti-CD39 antibody-drug conjugates against negative tumor cells.

DB is a human follicular lymphoma cell line which naturally expresses human CD39 on its cell surface, and is used for detecting the *in-vitro* killing activity effect of anti-CD39 antibody-drug conjugates against positive tumor cells.

MOLP-8 is a human multiple myeloma cell line which naturally expresses human CD39 on its cell surface, and is used for detecting the affinity activity and endocytosis activity of anti-CD39 antibody-drug conjugates on positive tumor cells.

Mino is a human mantle cell lymphoma cell line which naturally expresses human CD39 on its cell surface, and the cells are inoculated to CB17/SCID mice to establish a mantle cell lymphoma model for detecting the *in-vivo* anti-tumor effects of anti-CD39 antibody-drug conjugates.

### Meanings of abbreviations:

Linker component:

The structural formula of MC is

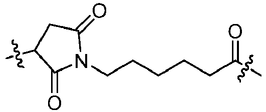

The structural formula of Val-Cit or "VC" is

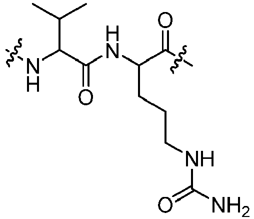

The structural formula of PAB is

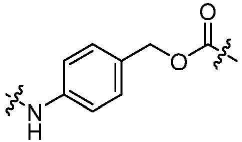

The structural formula of SPDB is

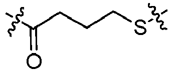

The structural formula of SMCC is

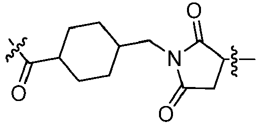

The structural formula of C4 is

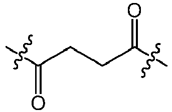

The structural formula of C5 is

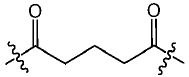

The structural formula of C6 is

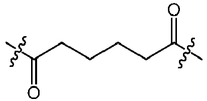

### Cytotoxic drugs:

MMAE = monomethyl auristatin E (MW 718)

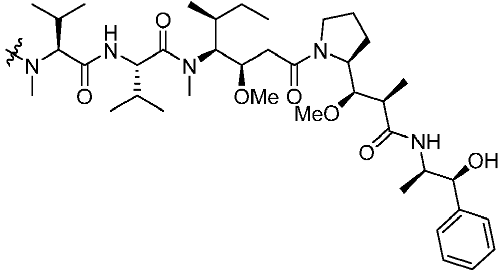

MMAF = auristatin E (MMAE) variant with phenylalanine (MW731.5) at the C-terminus of the drug

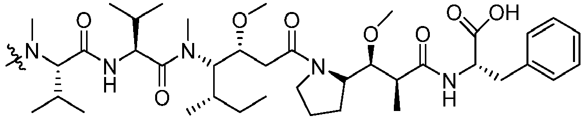

DM1 = N(2')-deacetyl-N(2')-(3-mercapto-1-oxopropyl)-maytansine

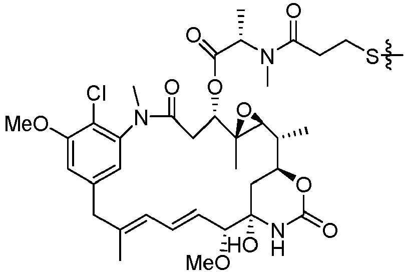

DM4 = N(2')-deacetyl-N2-(4-mercapto-4-methyl-1 -oxopentyl)-maytansine

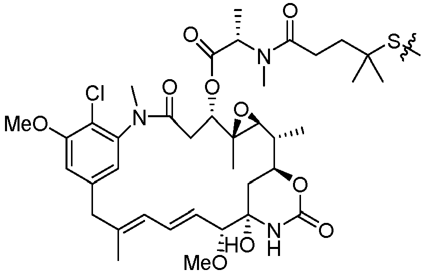

### Other structures:

The structure of NC is

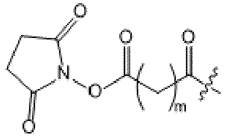

The structure of PC is

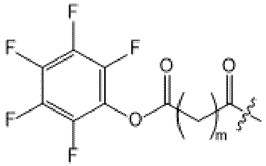

### II. Examples

The present invention is further described below with reference to examples, but these examples are not intended to limit the scope of the present invention.

Experimental procedures in which specific conditions are not indicated in examples and test examples of the present invention are generally conducted according to conventional conditions, or according to conditions recommended by the manufacturers of the starting materials or commercial products, see Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

The DNA sequences encoding the CDRs, variable regions, or light and heavy chains of the anti-CD39 antibody involved in the present invention can be designed according to corresponding amino acid sequences, which is a conventional technology in the art.

### Example 1. Preparation of Monoclonal Antibodies

### 1.1. Preparation of novel antibodies of the present application

By using a molecular cloning technology, firstly, cDNAs of amino acid sequences of the antibodies provided by the present application (as shown in Table 1, all antibodies in the table comprise the same antibody CDR sequences) were each cloned into an expression vector pcDNA3.4 containing a signal peptide. Then, plasmids respectively containing cDNA sequences of heavy chains and light chains of corresponding antibodies were amplified in *E. coli,* and subsequently, the expression plasmids containing the heavy chains and the light chains were co-transfected into CHO-S cells. The supernatant was harvested after culture and purified with MabSelect PrismA to obtain the monoclonal antibodies provided by the present application. By verification, the sequences of the antibodies were consistent with the sequences in the table below.

**Table 1: Amino acid sequences of the antibodies of the present application**

| Sequence No. | Sequence name | Amino acid sequence |
|---|---|---|
| SEQ ID NO:1 | HCDR1 | NYWMY |
| SEQ ID NO:2 | HCDR2 | RIAPSSGATKYNEKFKN |
| SEQ ID NO:3 | HCDR3 | SGIYYDYDWFFDV |
| SEQ ID NO:4 | LCDR1 | KASQDINKYIG |
| SEQ ID NO:5 | LCDR2 | YTSTLQP |
| SEQ ID NO:6 | LCDR3 | LQYHALLFT |
| SEQ ID NO:7 | VH of murine monoclonal antibody mF03 | QVQLQQPGAELVKPGASVKLSCKTSGYTFTNYWMY WVKHRPGRGLEWIGRIAPSSGATKYNEKFKNKATLT VDKPSSTAYMQLSSLTSEDSAVYYCARSGIYYDYDW FFDVSGTGTTVTVSS |
| SEQ ID NO:8 | VL of murine monoclonal antibody mF03 | DIQMTQSPSSLSASLGGKVTITCKASQDINKYIGWYQ HAPGKGPRLLIHYTSTLQPGIPSRFSGSGSGRDYSLSI SNLEPEDIATYYCLQYHALLFTFGSGTKLELK |
| SEQ ID NO:9 | Heavy chain of chimeric | QVQLQQPGAELVKPGASVKLSCKTSGYTFTNYWMY WVKHRPGRGLEWIGRIAPSSGATKYNEKFKNKATLT |
| | antibody F03 | VDKPSSTAYMQLSSLTSEDSAVYYCARSGIYYDYDW FFDVSGTGTTVTVSSASTKGPSVFPLAPCSRSTSESTA ALGCLVKDYFPEPVTVSWNSGALTSGVHTFPAVLQS SGLYSLSSVVTVPSSSLGTKTYTCNVDHKPSNTKVD KRVESKYGPPCPPCPAPEFLGGPSVFLFPPKPKDTLMI SRTPEVTCVVVDVSQEDPEVQFNWYVDGVEVHNAK TKPREEQFNSTYRVVSVLTVLHQDWLNGKEYKCKV SNKGLPSSIEKTISKAKGQPREPQVYTLPPSQEEMTK NQVSLTCLVKGFYPSDIAVEWESNGQPENNYKTTPP VLDSDGSFFLYSRLTVDKSRWQEGNVFSCSVMHEAL HNHYTQKSLSLSLGK |
| SEQ ID NO:10 | Light chain of chimeric antibody F03 | DIQMTQSPSSLSASLGGKVTITCKASQDINKYIGWYQ HAPGKGPRLLIHYTSTLQPGIPSRFSGSGSGRDYSLSI SNLEPEDIATYYCLQYHALLFTFGSGTKLELKRTVAA PSVFIFPPSDEQLKSGTASVVCLLNNFYPREAKVQWK VDNALQSGNSQESVTEQDSKDSTYSLSSTLTLSKAD YEKHKVYACEVTHQGLSSPVTKSFNRGEC |
| SEQ ID NO:11 | VH of humanized monoclonal antibody F306 | QVQLVQSGAEVKKPGASVKVSCKASGYTFTNYWM YWVRQAPGQGLEWMGRIAPSSGATKYNEKFKNRVT MTRDTSISTAYMELSRLRSDDTAVYYCARSGIYYDY DWFFDVWGQGTTVTVSS |
| SEQ ID NO:16 | VL of humanized monoclonal antibody F306 | DIQMTQSPSSLSASVGDRVTITCKASQDINKYIGWYQ QKPGKAPKLLIHYTSTLQPGVPSRFSGSGSGTDFTFTI SSLQPEDIATYYCLQYHALLFTFGQGTKLEIK |
| SEQ ID NO:12 | VH of humanized monoclonal antibody F307 | QVQLVQSGAEVKKPGASVKVSCKASGYTFTNYWM YWVRQAPGQGLEWMGRIAPSSGATKYNEKFKNRVT LTVDTSISTAYMELSRLRSDDTAVYYCARSGIYYDYD WFFDVWGQGTTVTVSS |
| SEQ ID NO:16 | VL of humanized monoclonal antibody F307 | DIQMTQSPSSLSASVGDRVTITCKASQDINKYIGWYQ QKPGKAPKLLIHYTSTLQPGVPSRFSGSGSGTDFTFTI SSLQPEDIATYYCLQYHALLFTFGQGTKLEIK |
| SEQ ID NO:13 | VH of humanized monoclonal antibody F308 | QVQLVQSGAEVKKPGASVKVSCKASGYTFTNYWM YWVRQAPGQGLEWIGRIAPSSGATKYNEKFKNRATL TVDTSISTAYMELSRLRSDDTAVYYCARSGIYYDYD WFFDVWGQGTTVTVSS |
| SEQ ID NO:16 | VL of humanized monoclonal antibody F308 | DIQMTQSPSSLSASVGDRVTITCKASQDINKYIGWYQ QKPGKAPKLLIHYTSTLQPGVPSRFSGSGSGTDFTFTI SSLQPEDIATYYCLQYHALLFTFGQGTKLEIK |
| SEQ ID NO:14 | VH of humanized monoclonal antibody F309 | QVQLVQSGAEVKKPGASVKVSCKASGYTFTNYWM YWVRQRPGRGLEWIGRIAPSSGATKYNEKFKNRATL TVDTSISTAYMELSRLRSDDTAVYYCARSGIYYDYD WFFDVWGQGTTVTVSS |
| SEQ ID NO:16 | VL of humanized monoclonal antibody F309 | DIQMTQSPSSLSASVGDRVTITCKASQDINKYIGWYQ QKPGKAPKLLIHYTSTLQPGVPSRFSGSGSGTDFTFTI SSLQPEDIATYYCLQYHALLFTFGQGTKLEIK |
| SEQ ID NO:15 | VH of humanized monoclonal antibody F310 | QVQLVQSGAEVKKPGASVKVSCKASGYTFTNYWM YWVRQRPGRGLEWIGRIAPSSGATKYNEKFKNRATL TVDTSISTAYMELSRLRSDDTAVYYCARSGIYYDYD WFFDVSGQGTTVTVSS |
| SEQ ID NO:16 | VL of humanized monoclonal antibody F310 | DIQMTQSPSSLSASVGDRVTITCKASQDINKYIGWYQ QKPGKAPKLLIHYTSTLQPGVPSRFSGSGSGTDFTFTI SSLQPEDIATYYCLQYHALLFTFGQGTKLEIK |
| SEQ ID NO:11 | VH of humanized monoclonal antibody F311 | QVQLVQSGAEVKKPGASVKVSCKASGYTFTNYWM YWVRQAPGQGLEWMGRIAPSSGATKYNEKFKNRVT MTRDTSISTAYMELSRLRSDDTAVYYCARSGIYYDY DWFFDVWGQGTTVTVSS |
| SEQ ID NO:17 | VL of humanized monoclonal antibody F311 | DIQMTQSPSSLSASVGDRVTITCKASQDINKYIGWYQ QKPGKAPKLLIHYTSTLQPGVPSRFSGSGSGRDYTFT ISSLQPEDIATYYCLQYHALLFTFGQGTKLEIK |
| SEQ ID NO:12 | VH of | QVQLVQSGAEVKKPGASVKVSCKASGYTFTNYWM |
| | humanized monoclonal antibody F312 | YWVRQAPGQGLEWMGRIAPSSGATKYNEKFKNRVT LTVDTSISTAYMELSRLRSDDTAVYYCARSGIYYDYD WFFDVWGQGTTVTVSS |
| SEQ ID NO: 17 | VL of humanized monoclonal antibody F312 | DIQMTQSPSSLSASVGDRVTITCKASQDINKYIGWYQ QKPGKAPKLLIHYTSTLQPGVPSRFSGSGSGRDYTFT ISSLQPEDIATYYCLQYHALLFTFGQGTKLEIK |
| SEQ ID NO: 13 | VH of humanized monoclonal antibody F313 | QVQLVQSGAEVKKPGASVKVSCKASGYTFTNYWM YWVRQAPGQGLEWIGRIAPSSGATKYNEKFKNRATL TVDTSISTAYMELSRLRSDDTAVYYCARSGIYYDYD WFFDVWGQGTTVTVSS |
| SEQ ID NO: 17 | VL of humanized monoclonal antibody F313 | DIQMTQSPSSLSASVGDRVTITCKASQDINKYIGWYQ QKPGKAPKLLIHYTSTLQPGVPSRFSGSGSGRDYTFT ISSLQPEDIATYYCLQYHALLFTFGQGTKLEIK |
| SEQ ID NO:14 | VH of humanized monoclonal antibody F314 | QVQLVQSGAEVKKPGASVKVSCKASGYTFTNYWM YWVRQRPGRGLEWIGRIAPSSGATKYNEKFKNRATL TVDTSISTAYMELSRLRSDDTAVYYCARSGIYYDYD WFFDVWGQGTTVTVSS |
| SEQ ID NO: 17 | VL of humanized monoclonal antibody F314 | DIQMTQSPSSLSASVGDRVTITCKASQDINKYIGWYQ QKPGKAPKLLIHYTSTLQPGVPSRFSGSGSGRDYTFT ISSLQPEDIATYYCLQYHALLFTFGQGTKLEIK |
| SEQ ID NO:15 | VH of humanized monoclonal antibody F315 | QVQLVQSGAEVKKPGASVKVSCKASGYTFTNYWM YWVRQRPGRGLEWIGRIAPSSGATKYNEKFKNRATL TVDTSISTAYMELSRLRSDDTAVYYCARSGIYYDYD WFFDVSGQGTTVTVSS |
| SEQ ID NO: 17 | VL of humanized monoclonal antibody F315 | DIQMTQSPSSLSASVGDRVTITCKASQDINKYIGWYQ QKPGKAPKLLIHYTSTLQPGVPSRFSGSGSGRDYTFT ISSLQPEDIATYYCLQYHALLFTFGQGTKLEIK |
| SEQ ID NO:18 | Heavy chain constant regions | ASTKGPSVFPLAPCSRSTSESTAALGCLVKDYFPEPV TVSWNSGALTSGVHTFPAVLQSSGLYSLSSVVTVPSS |
| | of humanized monoclonal antibodies F306, F307, F308, F309, F310, F311, F312, F313, F314 and F315 | SLGTKTYTCNVDHKPSNTKVDKRVESKYGPPCPPCP APEFLGGPSVFLFPPKPKDTLMISRTPEVTCVVVDVS QEDPEVQFNWYVDGVEVHNAKTKPREEQFNSTYRV VSVLTVLHQDWLNGKEYKCKVSNKGLPSSIEKTISK AKGQPREPQVYTLPPSQEEMTKNQVSLTCLVKGFYP SDIAVEWESNGQPENNYKTTPPVLDSDGSFFLYSRLT VDKSRWQEGNVFSCSVMHEALHNHYTQKSLSLSLG K |
| SEQ ID NO:19 | Light chain constant regions of humanized monoclonal antibodies F306, F307, F308, F309, F310, F311, F312, F313, F314 and F315 | |

### 1.2. Preparation of monoclonal antibody F1

An antibody F1 was prepared with reference to Innate patent WO2019096900A1, wherein the amino acid sequence of the heavy chain is set forth in SEQ ID NO: 37, and the amino acid sequence of the light chain is set forth in SEQ ID NO: 38. Because other anti-CD39 ADCs have not been disclosed in the prior art, the present invention used the F1 monoclonal antibody with the linker and the toxin used in the present invention to prepare ADC as a positive control group.

### Example 2. Obtainment of Cytotoxin Molecules

### 2.1. MC-VC-PAB-MMAE

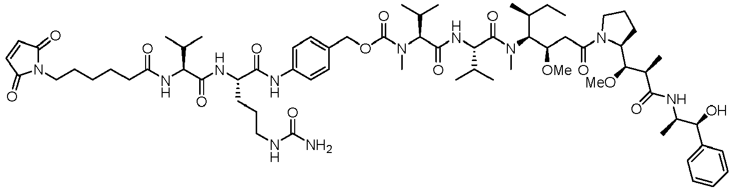

Commercially available (Cat. No. SET0201, Levena Biopharma).

### 2.2. MC-MMAF

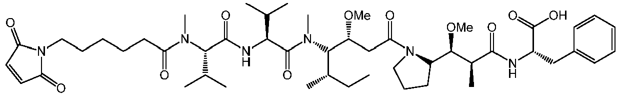

Commercially available (Cat. No. SET0202, Levena Biopharma).

### 2.3. SMCC-DM1

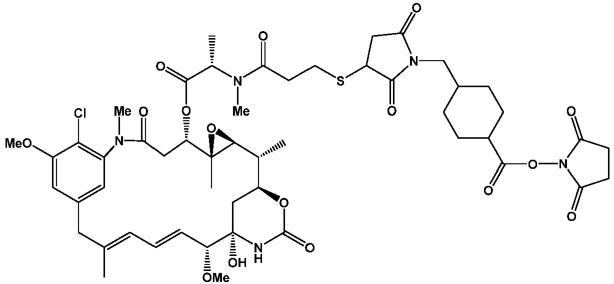

Commercially available (Cat. No. SET0101, Levena Biopharma).

### 2.4. SPDB-DM4

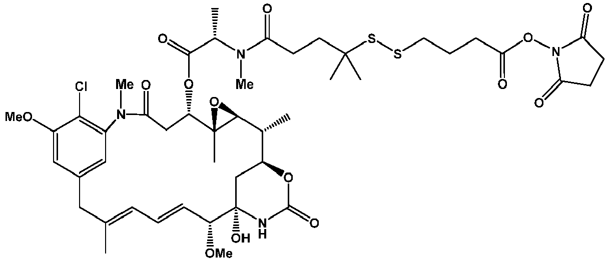

Commercially available (Cat. No. SET0102, Levena Biopharma).

### 2.5. Synthesis of VC-PAB-MMAE

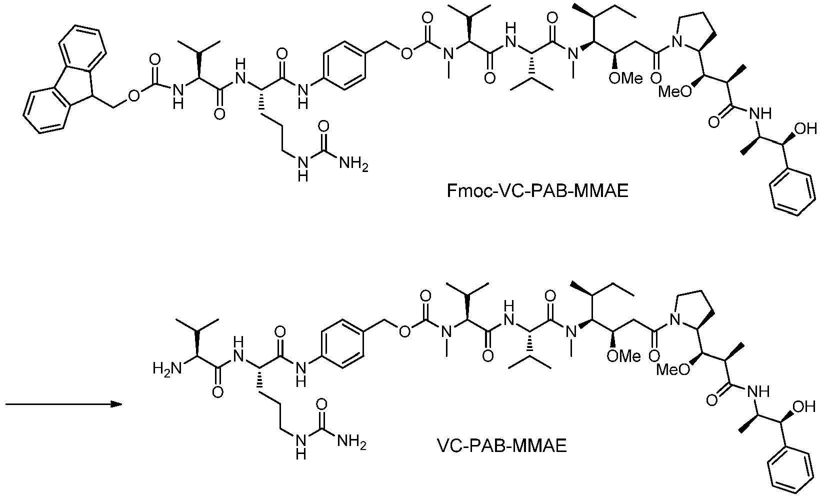

Fmoc-VC-PAB-MMAE was prepared according to the method disclosed in Example 18 of patent WO 2004/010957. Fmoc was stirred in DMF using 20% piperidine for 20 min and then removed, and a pure product of VC-PAB-MMAE was obtained by HPLC.

### 2.6. Synthesis of NC4-VC-PAB-MMAE

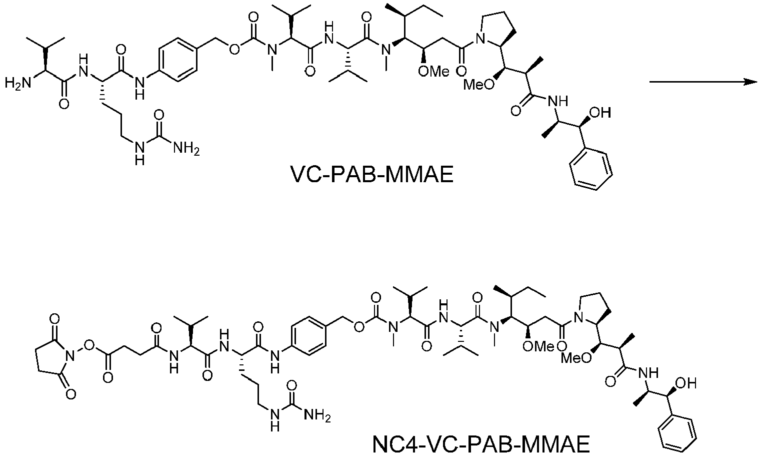

VC-PAB-MMAE (0.18 mmol) was dissolved in 1 mL of anhydrous DMF (*N,N-*dimethylformamide), and then DIEA (*N, N*-diisopropylethylamine) (0.44 mmol) and succinic anhydride (0.21 mmol) were separately added. After the reaction solution was stirred at room temperature for 10 min, dichloromethane (1 mL), *N*-hydroxysuccinimide (0.89 mmol), and EDC (1-ethyl-(3-dimethylaminopropyl)carbodiimide) (0.89 mmol) were added. The reaction solution was stirred at room temperature for 30 min, then concentrated by rotary evaporation under reduced pressure to remove the solvent, and purified by HPLC to obtain NC4-vc-PAB-MMAE, m/z: 1320.8 [M+H+].

### 2.7. Synthesis of PC4-VC-PAB-MMAE

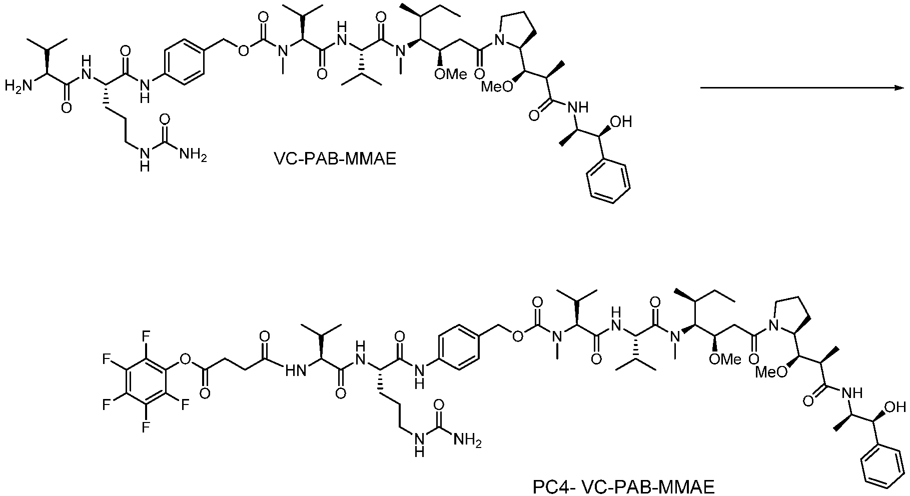

VC-PAB-MMAE (0.18 mmol) was dissolved in 1 mL of anhydrous DMF (*N,N-*dimethylformamide), and then DIEA (*N, N*-diisopropylethylamine, 0.44 mmol) and succinic anhydride (0.21 mmol) were separately added. After the reaction solution was stirred at room temperature for 10 min, dichloromethane (1 mL), pentafluorophenol (0.89 mmol), and EDC (0.89 mmol) were added. The reaction solution was stirred at room temperature for 30 min, then concentrated by rotary evaporation under reduced pressure to remove the solvent, and purified by HPLC to obtain PC4-VC-PAB-MMAE, m/z: 1389.9 [M+H+].

### 2.8. Synthesis of NC5-VC-PAB-MMAE

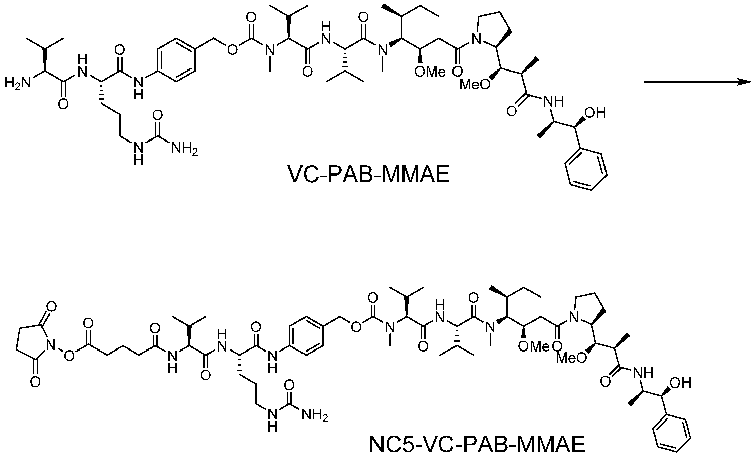

The synthesis in this example was conducted with reference to the synthesis of NC4-VC-PAB-MMAE in Example 2.6, but succinic anhydride was replaced with glutaric anhydride to obtain NC5-VC-PAB-MMAE, m/z: 1334.8 [M+H⁺].

### 2.9. Synthesis of PCS-VC-PAB-MMAE

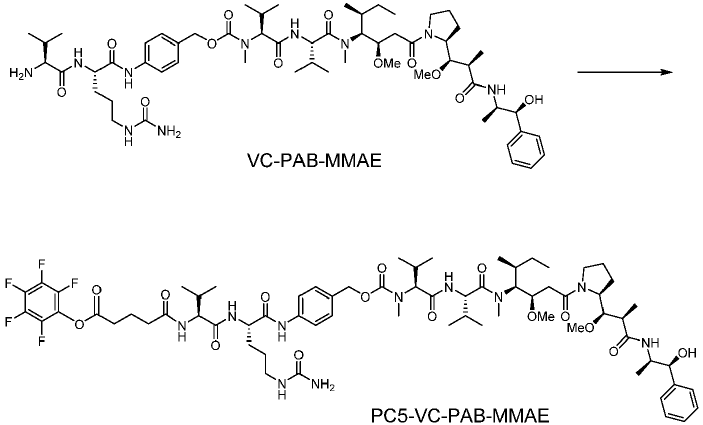

The synthesis in this example was conducted with reference to the synthesis of PC4-VC-PAB-MMAE in Example 2.7, but succinic anhydride was replaced with glutaric anhydride to obtain PC5-VC-PAB-MMAE, m/z: 1403.9 [M+H⁺].

### 2.10. Synthesis of NC6-VC-PAB-MMAE

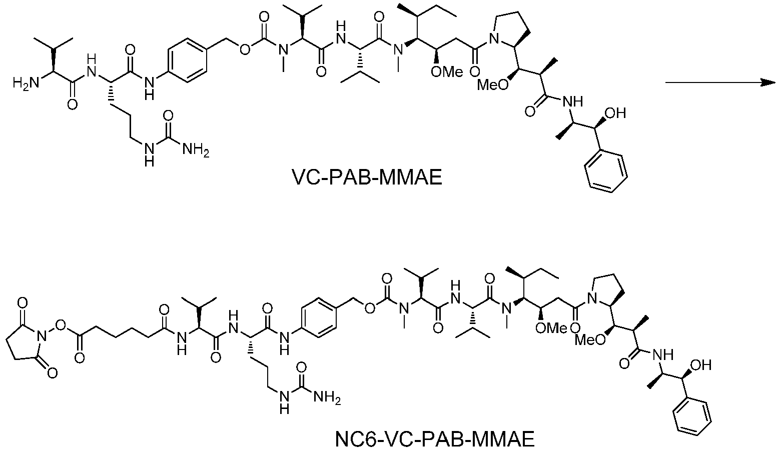

VC-PAB-MMAE (0.18 mmol) was dissolved in 1 mL of anhydrous DMF, and then DIEA (77 µL, 0.44 mmol) and adipic acid bis(N-hydroxysuccinimide) (1 mmol) were separately added. The reaction solution was stirred at room temperature for 20 min and then purified by HPLC to obtain NC6-VC-PAB-MMAE, m/z: 1348.8 [M+H⁺].

### 2.11. Synthesis of PC6-VC-PAB-MMAE

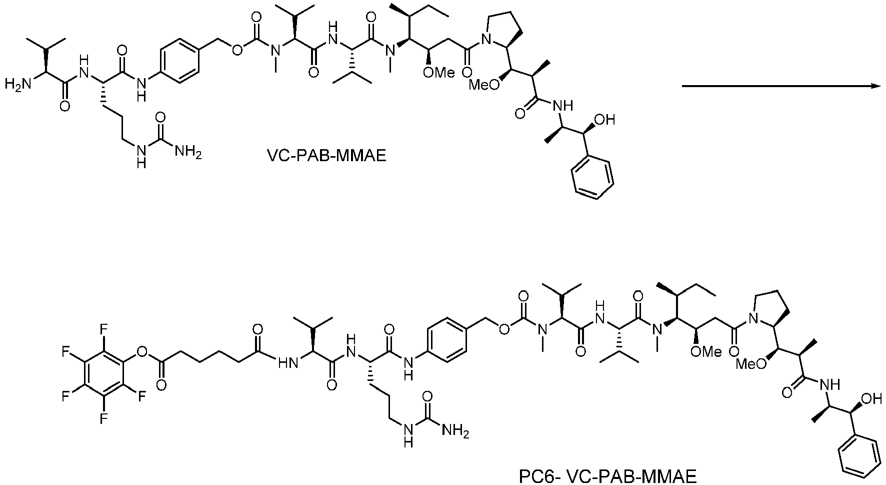

The synthesis in this example was conducted with reference to the synthesis of NC6-VC-PAB-MMAE in Example 2.10, but adipic acid bis(N-hydroxysuccinimide) was replaced with adipic acid bis(pentafluorophenyl) ester (synthesized according to the method in the literature: Liu, Yijiang, et al., Biomacromolecules (2015), 16(12), 3995-4003) to obtain PC6-VC-PAB-MMAE, m/z: 1417.7 [M+H⁺].

**Example 3. Preparation of antibody-drug conjugates** The amino acid sequences of variable and constant regions of heavy and light chains of F314 antibody in the examples below are shown in Table 1.

### 3.1. Preparation of F314-MC-VC-PAB-MMAE

Antibody reduction: F314 antibody was dissolved in PBS (pH 7±0.2) buffer solution at a concentration of 1-10 mg/mL, 3- to 10-fold molar equivalents of TCEP was then added, and the reaction solution was incubated at 4 °C-37 °C for 2-24 h.

Conjugation: A cytotoxin molecule MC-VC-PAB-MMAE was dissolved in DMA (*N*,*N*-dimethylacetamide) at a concentration of 10 mg/mL. After complete dissolution, 6- to 10-fold molar equivalents of MC-VC-PAB-MMAE was added to the reduced antibody solution for conjugation, with the final volume content of DMA controlled to be about 5%, and the mixture was allowed to react at 37 °C for 0.5-5 h. The DAR of the reaction product was monitored by HIC-HPLC within this time range, and the reaction was terminated when the DAR approached the target DAR of about 4.0 (since the DAR value of the product ADC changed along with the reaction time, the endpoint of the reaction was determined by monitoring the reaction product through HIC-HPLC, based on the DAR of the target product ADC).

Product purification: An excess of cysteine was added to the reaction solution, and the mixture was left to stand at room temperature for 30-60 min. Buffer exchange was performed on the reaction system by using an ultrafiltration tube with a molecular weight cut-off of 50 kD, and at the same time, DMA and unreacted cytotoxin molecules were removed. The final conjugate product was stored in PBS (pH 7±0.2) buffer solution. The average DAR value of the conjugate F314-MC-VC-PAB-MMAE was detected to be 4.0 by the HIC-HPLC method.

The structure of the conjugate F314-MC-VC-PAB-MMAE is shown in the following formula: 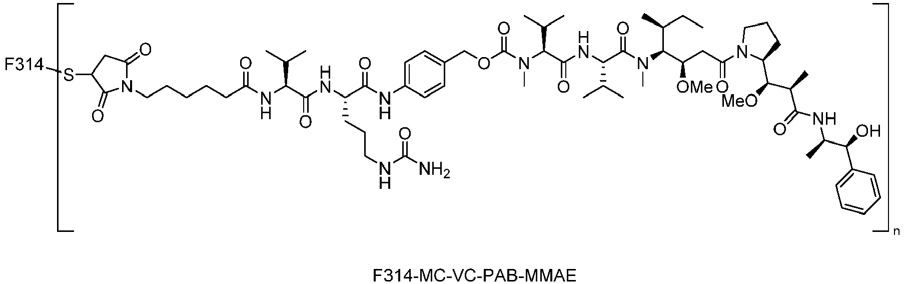 wherein the average DAR value n was 4.0.

The calculation formula for the average DAR value is as follows: average DAR value = (0 × DAR0 area percentage (%) + 2 × DAR2 area percentage (%) + 4 × DAR4 area percentage (%) + 6 × DAR6 area percentage (%) + 8 × DAR8 area percentage (%)) / 100

The calculation results are as follows:

| HIC-HPLC area percentage (%) | | | | | Average DAR value |
|---|---|---|---|---|---|
| DAR0 | DAR2 | DAR4 | DAR6 | DAR8 | |
| 7.68 | 14.84 | 51.77 | 20.60 | 5.14 | 4.0 |

### 3.2. Preparation of F314-MC-MMAF

The preparation in this example was conducted with reference to the preparation of F314-MC-VC-PAB-MMAE in Example 3.1, but the cytotoxin molecule MC-VC-PAB-MMAE was replaced with MC-MMAF. The average DAR value of the conjugate F314-MC-MMAF was detected to be 1.5 using Ellman's Reagent (for the detection method and the calculation method for the average DAR value, refer to the literature: Interaction of Nitric Oxide with 2-Thio-5-nitrobenzoic Acid: Implications for the Determination of Free Sulfhydryl Groups by Ellman's Reagent).

The structure of the conjugate F314-MC-MMAF is shown in the following formula: 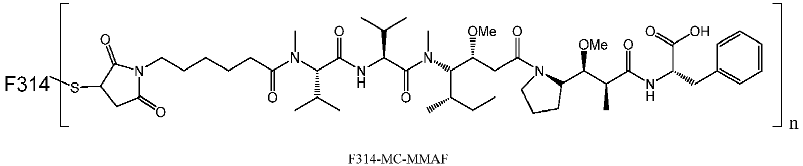 wherein the average DAR value n was 1.5.

### 3.3. Preparation of F314-SMCC-DM1

Conjugation: F314 antibody was dissolved in PBS (pH 7±0.2) buffer solution at a concentration of 1-10 mg/mL, and small-molecule SMCC-DM1 was dissolved in DMA at a concentration of 10 mg/mL. After complete dissolution, 6- to 10-fold molar equivalents of SMCC-DM1 was added to the antibody solution for conjugation, with the final volume content of DMA controlled to be about 5%, and the mixture was allowed to react at room temperature in the dark for 12-24 h;

Product purification: Buffer exchange was performed on the reaction system by using an ultrafiltration tube with a molecular weight cut-off of 50 kD, and at the same time, DMA and unreacted cytotoxin molecules were removed. The final conjugate product was stored in PBS (pH 7±0.2) buffer solution. The average DAR value of F314-SMCC-DM1 was detected to be 5.0 (for the detection method and the calculation method for the average DAR value, refer to the literature: yan chen, Drug-to-Antibody Ratio (DAR) by UV/Vis Spectroscopy).

The structure of the conjugate F314-SMCC-DM1 is shown in the following formula: 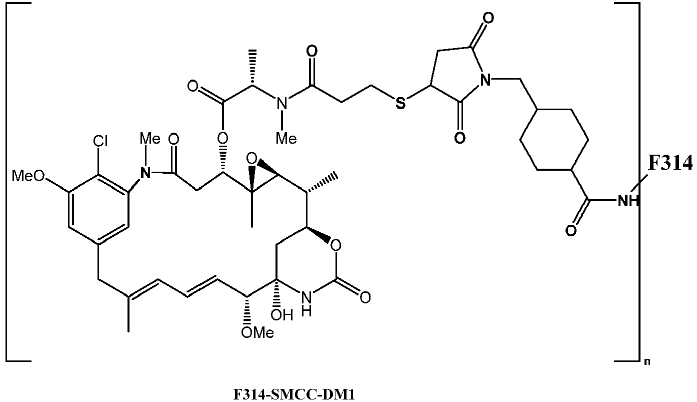 wherein the average DAR value n was 5.0.

### 3.4. Preparation of F314-SPDB-DM4

The preparation in this example was conducted with reference to the preparation of F314-SMCC-DM1 in Example 3.3, but the cytotoxin molecule SMCC-DM1 was replaced with SPDB-DM4. The DAR value of F314-SPDB-DM4 was detected to be 3.8.

The structure of the conjugate F314-SPDB-DM4 is shown in the following formula: 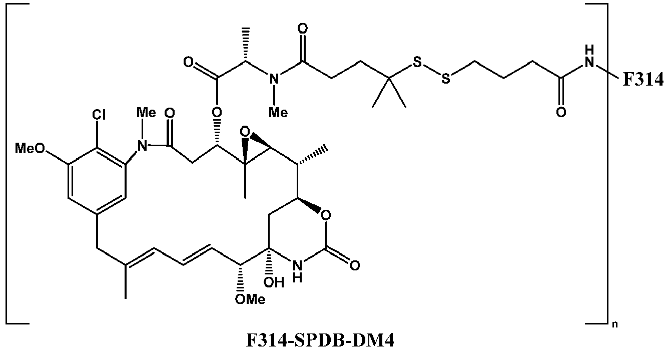 wherein the average DAR value n was 3.8.

### 3.5. Preparation of F314-C4-VC-PAB-MMAE

F314 antibody was dissolved in PBS (pH 7±0.2) buffer solution at a concentration of 1-10 mg/mL, and NC4-VC-PAB-MMAE or PC4-VC-PAB-MMAE was dissolved in DMA at a concentration of 10 mg/mL. After complete dissolution, 6- to 10-fold molar equivalents of NC4-VC-PAB-MMAE or PC4-VC-PAB-MMAE solution was added to the antibody solution for conjugation, with the final volume content of DMA controlled to be about 5%, and the mixture was allowed to react for 2-24 h at room temperature in the dark. The DAR of the reaction product was monitored by HIC-HPLC within this time range, and the reaction was terminated when the DAR approached the target DAR (since the DAR value of the product ADC changed along with the reaction time, the endpoint of the reaction was determined by monitoring the reaction product through HIC-HPLC, based on the DAR of the target product ADC). Buffer exchange was performed on the reaction system by using an ultrafiltration tube with a molecular weight cut-off of 50 kD, and at the same time, DMA and unreacted cytotoxin molecules were removed. The final conjugate product was stored in PBS (pH 7±0.2) buffer solution. The average DAR value of the conjugate F314-C4-VC-PAB-MMAE was detected to be 2.1 by the HIC-HPLC method.

The structure of the conjugate F314-C4-VC-PAB-MMAE is shown in the following formula: 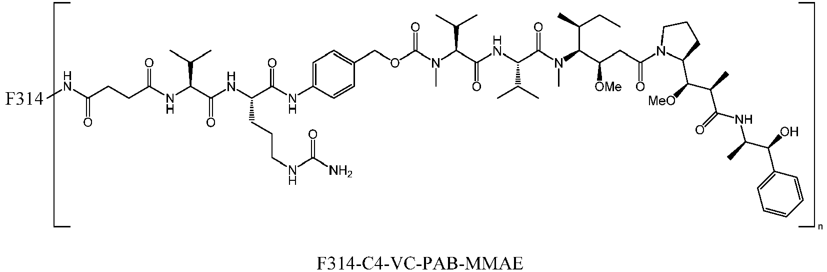 wherein the average DAR value n was 2.1.

The calculation formula for the average DAR value is as follows: average DAR value = (0 × DAR0 area percentage (%) +1 × DAR1 area percentage (%) +2 × DAR2 area percentage (%)+3 × DAR3 area percentage (%) +4 × DAR4 area percentage (%)) / 100

The calculation results are as follows:

| HIC-HPLC area percentage (%) | | | | | Average DAR value |
|---|---|---|---|---|---|
| DAR0 | DAR1 | DAR2 | DAR3 | DAR4 | |
| 3.54 | 24.10 | 43.98 | 15.30 | 13.07 | 2.1 |

### 3.6. Preparation of F314-C5-VC-PAB-MMAE

The preparation in this example was conducted with reference to the preparation of F314-C4-VC-PAB-MMAE in Example 3.5, but NC5-VC-PAB-MMAE or PC5-VC-PAB-MMAE was used as the cytotoxin molecule, and meanwhile, 2-, 6-, 7.5- and 10-fold molar equivalents of the cytotoxin molecule were added to the antibody solution for conjugation to prepare products with average DAR values of 0.8, 1.8, 2.3 and 3.0, respectively.

The structure of the conjugate F314-C5-VC-PAB-MMAE is shown in the following formula: 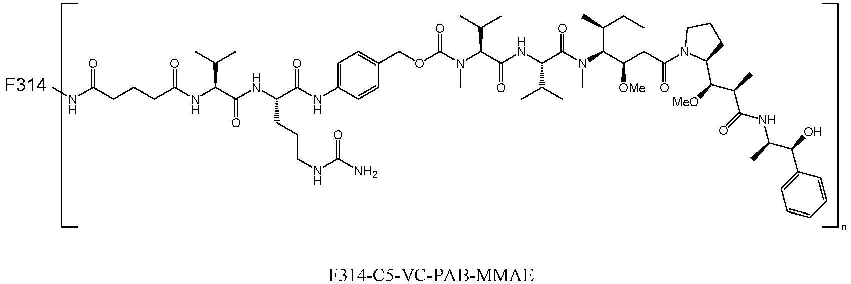 wherein the average DAR value n was 0.8, 1.8, 2.3, or 3.0.

The calculation formula for the average DAR value is as follows: average DAR value = (0 × DAR0 area percentage (%) +1 × DAR1 area percentage (%) +2 × DAR2 area percentage (%) +3 × DAR3 area percentage (%) +4 × DAR4 area percentage (%)) / 100

The calculation results are as follows:

| F314-C5-VC-PAB-MMAE with different average DAR values | HIC-HPLC area percentage (%) | | | | | Average DAR value |
|---|---|---|---|---|---|---|
| | DAR0 | DAR1 | DAR2 | DAR3 | DAR4 | |
| F314-C5-VC-PAB-MMAE #1 | 39.46 | 40.81 | 19.73 | 0 | 0 | 0.8 |
| F314-C5-VC-PAB-MMAE #2 | 7.58 | 31.18 | 39.63 | 15.79 | 5.83 | 1.8 |
| F314-C5-VC-PAB-MMAE #3 | 1.73 | 17.62 | 44.31 | 20.99 | 15.35 | 2.3 |
| F314-C5-VC-PAB-MMAE #4 | 0.48 | 6.46 | 29.31 | 22.72 | 41.03 | 3.0 |

The results of mass spectrometry are consistent with those of HIC-HPLC, and the molecular weight difference between each DAR component and a naked antibody (DAR0) as measured by mass spectrometry is as follows:

| Δ(DAR1-DAR0) | | Δ(DAR2-DAR0) | | Δ(DAR3-DAR0) | | Δ(DAR4-DAR0) | |
|---|---|---|---|---|---|---|---|
| Theoretical value | Measured value | Theoretical value | Measured value | Theoretical value | Measured value | Theoretical value | Measured value |
| 1220 | 1216 | 2440 | 2435 | 3660 | 3655 | 4880 | 4878 |

### 3.7. Preparation of F314-C6-VC-PAB-MMAE

The preparation in this example was conducted with reference to the preparation of F314-C4-VC-PAB-MMAE in Example 3.5, but NC6-VC-PAB-MMAE or PC6-VC-PAB-MMAE was used as the cytotoxin molecule. The average DAR value of the conjugate F314-C6-VC-PAB-MMAE was detected to be 2.1 by the HIC-HPLC method.

The structure of the conjugate F314-C6-VC-PAB-MMAE is shown in the following formula: 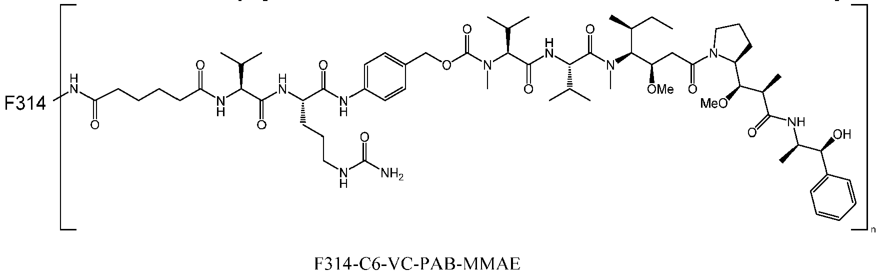 wherein the average DAR value n was 2.1.

The calculation formula for the average DAR value is as follows: average DAR value = (0 × DAR0 area percentage (%)+1 × DAR1 area percentage (%) +2 × DAR2 area percentage (%) +3 × DAR3 area percentage (%) +4 × DAR4 area percentage (%)) / 100

The calculation results are as follows:

| HIC-HPLC area percentage (%) | | | | | Average DAR value |
|---|---|---|---|---|---|
| DAR0 | DAR1 | DAR2 | DAR3 | DAR4 | |
| 3.65 | 22.58 | 43.34 | 16.49 | 13.94 | 2.1 |

### 3.8. Preparation of F1-SMCC-DM1

The preparation in this example was conducted with reference to the preparation of F314-SMCC-DM1, but the monoclonal antibody F314 was replaced with F1 to obtain F1-SMCC-DM1. The average DAR value n of F1-SMCC-DM1 was detected to be 5.5. 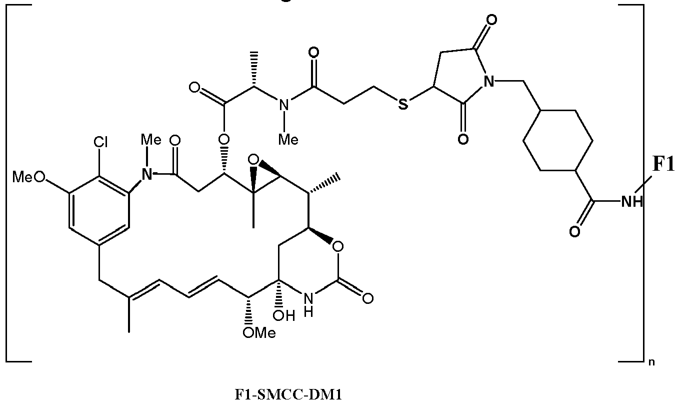 wherein the average DAR value n was 5.5.

### Example 4. In-Vitro Killing Activity of ADCs against Positive Tumor Cells

Each ADC or antibody sample to be tested was diluted to an initial concentration of 6.67 µg/mL with a 1640+10% FBS (Gibco) complete medium, then diluted in a 3-fold gradient to give a total of 5 concentration points, and added into a 96-well cell plate at 100 µL/well, and zero-concentration control wells (i.e., containing complete medium without the ADC or antibody sample to be tested) were set. DB cells (Nanjing Cobioer Biosciences Co., Ltd.) in logarithmic growth phase were collected, diluted to (0.8-1.2) × 10⁵ cells/mL with a 1640+10% FBS (Gibco) complete medium, and then added into the above 96-well plate at 100 µL/well. The 96-well plate was placed in a CO₂ incubator at 37 °C and left to stand for 60-96 h. CCK-8 (Dojindo Laboratories, CK04) was then added at 20 µL/well, and the mixture was incubated for 4-6 h of color development. The OD₄₅₀ₙₘ readings were then detected by using a multi-mode microplate reader (Molecular Devices). Calculation formula for cell viability (%): OD_{sample well}/OD_{control well} × 100%.

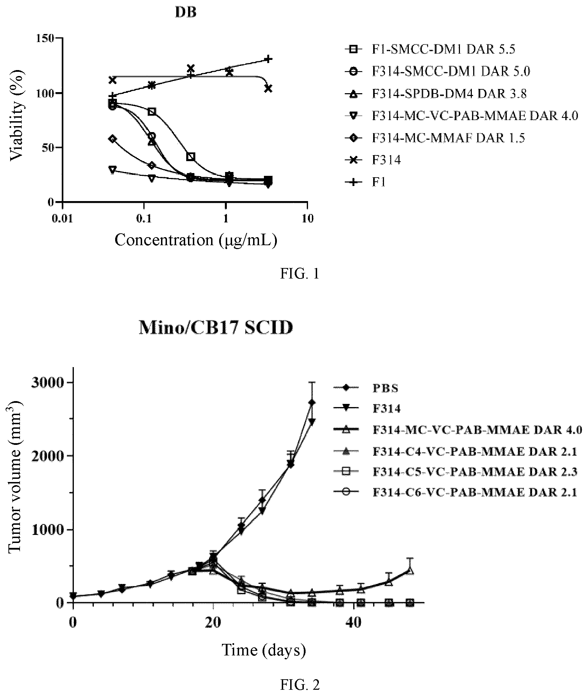

The results are shown in FIG. 1, indicating that for the DB tumor cells positive to antigen expression, the monoclonal antibodies F1 and F314 had no direct killing effect, the ADCs with different conjugated toxins all exhibited significant levels of killing effect, and the killing activity of F314-SMCC-DM1, F314-SPDB-DM4, F314-MC-VC-PAB-MMAE and F314-MC-MMAF was superior to that of F1-SMCC-DM1.

### Example 5. In-Vivo Anti-Tumor Activity of ADCs

5 × 10⁶ Mino cells (ATCC) (plus 50% Matrigel, Thermo Fisher) were inoculated into the right ventral side of CB17/SCID mice (Shanghai Slac Laboratory Animal Co., Ltd., aged 6-8 weeks). When the tumor volume reached the average value of about 90 mm³, the mice were divided into groups, and the day of grouping was recorded as Day 0. The blank control group and F314 group were subjected to administration starting on Day 0, with 5 animals in each group, wherein the administration was performed by intraperitoneal injection twice a week for 6 times consecutively. The dose for the F314 group was 10 mg/kg, and the blank control group was injected with an equal volume of PBS. The F314-MC-VC-PAB-MMAE group, F314-C4-VC-PAB-MMAE group, F314-C5-VC-PAB-MMAE group and F314-C6-VC-PAB-MMAE group were subjected to single administration on Day 17 (the mean tumor volume was about 450 mm³), with 4 animals in each group, wherein the administration was performed by intravenous injection, with the dose of being 88 µg/kg based on the small-molecule toxin MMAE. The length and width of the tumor were measured with a vernier caliper, and the tumor volume was calculated using the formula TV = (length × width²) / 2. The mean tumor volume for each group of mice was taken to plot the tumor growth curve. The results are shown in FIG. 2, indicating that under the conditions of this experiment, the F314 monoclonal antibody (calculated based on the mass of the F314 monoclonal antibody, the amount of the antibody in the F314 group was greater than those in the ADC administration groups) did not exhibit anti-tumor effect, whereas the 4 ADCs of the present invention exhibited significant anti-tumor effects, wherein tumors of all mice in the F314-C4-VC-PAB-MMAE, F314-C5-VC-PAB-MMAE, and F314-C6-VC-PAB-MMAE groups were completely regressed.

### Example 6. In-Vitro Killing Activity of ADCs against Negative Tumor Cells

Each ADC or antibody sample to be tested was diluted to an initial concentration of 6.67 µg/mL with McCoy's 5A+10% FBS complete medium, then diluted in a 3-fold gradient to give a total of 5 concentration points, and added into a 96-well cell plate at 100 µL/well, and zero-concentration control wells (i.e., containing complete medium without the ADC or antibody sample to be tested) were set. SKOV-3 cells (ATCC) in logarithmic growth phase were collected, diluted to (0.8-1.2) × 10⁵ cells/mL with McCoy's 5A +10% FBS complete medium, and then added into the above 96-well plate at 100 µL/well. The 96-well plate was placed in a CO₂ incubator at 37 °C and left to stand for 60-96 h. CCK-8 was then added at 20 µL/well, and the mixture was incubated for 4-6 h of color development. The OD_{450 nm} readings were then detected by using a multi-mode microplate reader. Calculation formula for cell viability (%): OD_{sample well}/OD_{controll well} × 100%.

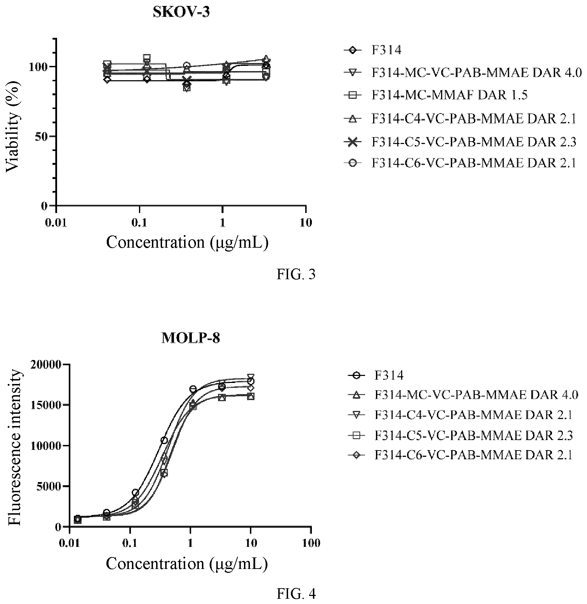

The results are shown in FIG. 3, indicating that for the SKOV-3 tumor cells that do not express human CD39, the F314 monoclonal antibody and its ADCs with different conjugated toxins (F314-MC-MMAF, F314-MC-VC-PAB-MMAE, F314-C4-VC-PAB-MMAE, F314-C5-VC-PAB-MMAE, and F314-C6-VC-PAB-MMAE) all exhibited no killing effect, demonstrating that the ADCs of the present invention had good targeting properties, and the ADCs were stable without causing non-specific killing due to the shedding of cytotoxin molecules.

### Example 7. Affinity Activity of ADCs for Positive Tumor Cells

Each ADC or antibody sample to be tested was diluted to an initial concentration of 20 µg/mL with 1 × PBS, then diluted in a 3-fold gradient to give a total of 7 concentration points, and added into a 96-well V-bottom plate at 50 µL/well, and meanwhile, zero-concentration control wells (i.e., containing complete medium without the ADC or antibody sample to be tested) were set. MOLP-8 cells (DSMZ) were collected, resuspended to 8.0 × 10⁶ cells/mL with 1 × PBS, and then added into the above 96-well plate at 50 µL/well. The mixture was well mixed and then incubated at 4 °C for 0.5 h. Washing: 1 × PBS was added at 100 µL/well, and the mixture was centrifuged at 2000 rpm for 7 min, followed by removal of the supernatant; 1 × PBS was further added at 200 µL/well, and the mixture was well mixed by pipetting and then centrifuged at 2000 rpm for 7 min, followed by removal of the supernatant. FITC-labeled goat anti-human H + L secondary antibody (Jackson 109-095-088) was diluted in a ratio of 1:100 and added at 100 µL/well, and the mixture was well mixed and then incubated at 4 °C for 0.5 h. The foregoing washing procedure was repeated. 1 × PBS was added at 200 µL/well to resuspend the cells, and the resuspended cells were analyzed on a flow cytometer.

The results are shown in FIG. 4, indicating that the F314 monoclonal antibody and the ADCs of the present invention all exhibited an affinity for MOLP-8 cells expressing CD39, and the affinity of the ADCs did not show significant change compared with the naked antibody (i.e., F314 monoclonal antibody).

### Example 8. Endocytosis Activity of ADCs

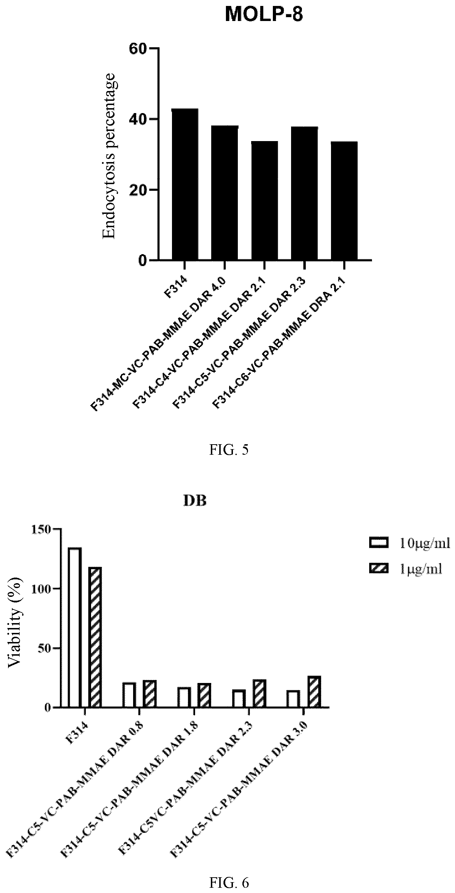

Each ADC or antibody sample to be tested was diluted to 20 µg/mL with 1 × PBS and then added to a 1.5 mL centrifuge tube at 100 µL/tube. MOLP-8 cells were collected, resuspended to 1.0 × 10⁷ cells/mL with 1 × PBS, and then added into the above 1.5 mL centrifuge tube at 100 µL/tube. The mixture was well mixed and then incubated at 4 °C for 0.5 h. Washing: 1 mL of 1 × PBS was added, and the mixture was well mixed by pipetting and centrifuged at 2000 rpm for 7min, followed by removal of the supernatant. The washing was repeated once. 200 µL of 1 × PBS was added to resuspend the cells, and the mixture was aliquoted into two aliquots, one of which was still left to stand at 4 °C and the other was incubated in a CO₂ incubator at 37 °C for 1 h. The foregoing washing procedure was repeated. FITC-labeled goat anti-human H + L secondary antibody (Jackson 109-095-088) was diluted in a ratio of 1:100 and added at 100 µL/tube, and the mixture was well mixed and then incubated at 4 °C for 0.5 h. The foregoing washing procedure was repeated. 1 × PBS was added at 200 µL/tube to resuspend the cells, and the resuspended cells were analyzed on a flow cytometer. Calculation formula for Endocytosis (%): (MFI_{4°C} - MFI_{37°C})/MFI_{4°C} × 100%. The results are shown in FIG. 5, indicating that the ADCs retained the endocytosis activity of the naked antibody.

### Example 9. In-Vitro Killing Activity of F314-C5-VC-PAB-MMAE with Different DAR Values against Positive Tumor Cells

Each ADC or antibody sample to be tested was diluted to an initial concentration of 20 µg/mL with a 1640+10% FBS complete medium, then diluted by 10 folds to 2 µg/mL, and added into a 96-well cell plate at 100 µL/well, and zero-concentration control wells (i.e., containing complete medium without the ADC or antibody sample to be tested) were set. DB cells in logarithmic growth phase were collected, diluted to (0.8-1.2) × 10⁵ cells/mL with a complete medium, and then added into the above 96-well plate at 100 µL/well. The 96-well plate was placed in a CO₂ incubator at 37 °C and left to stand for 60-96 h. CCK-8 was then added at 20 µL/well, and the mixture was incubated for 4-6 h of color development. The OD_{450 nm} readings were then detected by using a multi-mode microplate reader. Calculation formula for cell viability (%): OD_{sample well}/OD_{control well} × 100%.

The results are shown in FIG. 6, indicating that at the action concentrations of 10 µg/mL and 1 µg/mL, F314-C5-VC-PAB-MMAE with a DAR range of 0.8-3.0 had significantly improved killing activity against positive tumor cells compared with the naked antibody F314.

## Claims

1. An antibody-drug conjugate represented by general formula (I) or a pharmaceutically acceptable salt or solvate thereof: 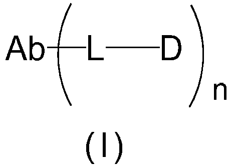 wherein
L is a linker;
D is a cytotoxin molecule;
"-" is a bond;
n is an average conjugation quantity of the cytotoxin molecule conjugated to the antibody, 0 < n ≤ 10, preferably 0.8 ≤ n ≤ 5.5, and n can be an integer or a non-integer;
Ab is an anti-CD39 antibody or an antigen-binding fragment thereof, preferably comprising the following CDRs:
an HCDR1, the amino acid sequence of which is set forth in SEQ ID NO: 1, or which comprises the amino acid sequence set forth in SEQ ID NO: 1;
an HCDR2, the amino acid sequence of which is set forth in SEQ ID NO: 2, or which comprises the amino acid sequence set forth in SEQ ID NO: 2;
an HCDR3, the amino acid sequence of which is set forth in SEQ ID NO: 3, or which comprises the amino acid sequence set forth in SEQ ID NO: 3;
an LCDR1, the amino acid sequence of which is set forth in SEQ ID NO: 4, or which comprises the amino acid sequence set forth in SEQ ID NO: 4;
an LCDR2, the amino acid sequence of which is set forth in SEQ ID NO: 5, or which comprises the amino acid sequence set forth in SEQ ID NO: 5; and
an LCDR3, the amino acid sequence of which is set forth in SEQ ID NO: 6, or which comprises the amino acid sequence set forth in SEQ ID NO: 6.

2. The antibody-drug conjugate or the pharmaceutically acceptable salt or solvate thereof according to claim 1, wherein the amino acid sequence of a heavy chain variable region of the anti-CD39 antibody or the antigen-binding fragment thereof is set forth in SEQ ID NO: 7, or has at least 90%, at least 95%, at least 96%, at least 97%, at least 98% or at least 99% sequence identity to SEQ ID NO: 7; and/or the amino acid sequence of a light chain variable region of the anti-CD39 antibody or the antigen-binding fragment thereof is set forth in SEQ ID NO: 8, or has at least 90%, at least 95%, at least 96%, at least 97%, at least 98% or at least 99% sequence identity to SEQ ID NO: 8.

3. The antibody-drug conjugate or the pharmaceutically acceptable salt or solvate thereof according to claim 1 or 2, wherein the anti-CD39 antibody or the antigen-binding fragment thereof is a murine antibody or an antigen-binding fragment thereof, further comprising a heavy chain constant region of a murine IgG1, IgG2, IgG3 or IgG4 or a variant thereof; and/or further comprising a light chain constant region of a murine κ or λ chain or a variant thereof; or
the anti-CD39 antibody or the antigen-binding fragment thereof is a chimeric antibody or an antigen-binding fragment thereof, further comprising a heavy chain constant region of a human IgG1, IgG2, IgG3 or IgG4 or a variant thereof, preferably comprising a human IgG4 heavy chain constant region; and/or further comprising a light chain constant region of a human κ or λ chain or a variant thereof, preferably a human κ light chain constant region;
preferably, the amino acid sequence of a heavy chain of the chimeric antibody is set forth in SEQ ID NO: 9, or has at least 90%, at least 95%, at least 96%, at least 97%, at least 98% or at least 99% sequence identity to SEQ ID NO: 9; and/or the amino acid sequence of a light chain of the antibody is set forth in SEQ ID NO: 10, or has at least 90%, at least 95%, at least 96%, at least 97%, at least 98% or at least 99% sequence identity to SEQ ID NO: 10.

4. The antibody-drug conjugate or the pharmaceutically acceptable salt or solvate thereof according to claim 1, wherein the anti-CD39 antibody or the antigen-binding fragment thereof is a humanized antibody or an antigen-binding fragment thereof, further comprising a heavy chain FR of a human IgG1, IgG2, IgG3 or IgG4 or a variant thereof, preferably comprising an FR of a human germline heavy chain IGHV1-2*02 or a variant thereof; and/or further comprising a light chain FR of a human κ or λ chain or a variant thereof, preferably an FR of a human germline light chain IGKV1-33*01 or a variant thereof;
preferably, the amino acid sequence of a heavy chain variable region of the humanized antibody or the antigen-binding fragment thereof is set forth in SEQ ID NO: 11, 12, 13, 14 or 15, or has at least 90%, at least 95%, at least 96%, at least 97%, at least 98% or at least 99% sequence identity to SEQ ID NO: 11, 12, 13, 14 or 15; and/or the amino acid sequence of a light chain variable region of the humanized antibody or the antigen-binding fragment thereof is set forth in SEQ ID NO: 16 or 17, or has at least 90%, at least 95%, at least 96%, at least 97%, at least 98% or at least 99% sequence identity to SEQ ID NO: 16 or 17;
wherein preferably, the amino acid sequence of the heavy chain variable region of the humanized antibody or the antigen-binding fragment thereof is set forth in SEQ ID NO: 11, or has at least 90%, at least 95%, at least 96%, at least 97%, at least 98% or at least 99% sequence identity to SEQ ID NO: 11; and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 12, or has at least 90%, at least 95%, at least 96%, at least 97%, at least 98% or at least 99% sequence identity to SEQ ID NO: 12; and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 13, or has at least 90%, at least 95%, at least 96%, at least 97%, at least 98% or at least 99% sequence identity to SEQ ID NO: 13; and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 14, or has at least 90%, at least 95%, at least 96%, at least 97%, at least 98% or at least 99% sequence identity to SEQ ID NO: 14; and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 15, or has at least 90%, at least 95%, at least 96%, at least 97%, at least 98% or at least 99% sequence identity to SEQ ID NO: 15; and the amino acid sequence of the light chain variable region of the humanized antibody or the antigen-binding fragment thereof is set forth in SEQ ID NO: 16, or has at least 90%, at least 95%, at least 96%, at least 97%, at least 98% or at least 99% sequence identity to SEQ ID NO: 16; or
the amino acid sequence of the heavy chain variable region of the humanized antibody or the antigen-binding fragment thereof is set forth in SEQ ID NO: 11, or has at least 90%, at least 95%, at least 96%, at least 97%, at least 98% or at least 99% sequence identity to SEQ ID NO: 11; and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17; or
the amino acid sequence of the heavy chain variable region of the humanized antibody or the antigen-binding fragment thereof is set forth in SEQ ID NO: 12, or has at least 90%, at least 95%, at least 96%, at least 97%, at least 98% or at least 99% sequence identity to SEQ ID NO: 12; and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17; or
the amino acid sequence of the heavy chain variable region of the humanized antibody or the antigen-binding fragment thereof is set forth in SEQ ID NO: 13, or has at least 90%, at least 95%, at least 96%, at least 97%, at least 98% or at least 99% sequence identity to SEQ ID NO: 13; and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17; or
the amino acid sequence of the heavy chain variable region of the humanized antibody or the antigen-binding fragment thereof is set forth in SEQ ID NO: 14, or has at least 90%, at least 95%, at least 96%, at least 97%, at least 98% or at least 99% sequence identity to SEQ ID NO: 14; and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17; or
the amino acid sequence of the heavy chain variable region of the humanized antibody or the antigen-binding fragment thereof is set forth in SEQ ID NO: 15, or has at least 90%, at least 95%, at least 96%, at least 97%, at least 98% or at least 99% sequence identity to SEQ ID NO: 15; and the amino acid sequence of the light chain variable region of the humanized antibody or the antigen-binding fragment thereof is set forth in SEQ ID NO: 17, or has at least 90%, at least 95%, at least 96%, at least 97%, at least 98% or at least 99% sequence identity to SEQ ID NO: 17.

5. The antibody-drug conjugate or the pharmaceutically acceptable salt or solvate thereof according to claim 4, wherein the humanized antibody or the antigen-binding fragment thereof further comprises a heavy chain constant region of the human IgG1, IgG2, IgG3 or IgG4 or the variant thereof, preferably comprises a human IgG4 heavy chain constant region, wherein more preferably, the amino acid sequence of the human IgG4 heavy chain constant region is set forth in SEQ ID NO: 18, or has at least 90%, at least 95%, at least 96%, at least 97%, at least 98% or at least 99% sequence identity to SEQ ID NO: 18; and/or the humanized antibody or the antigen-binding fragment thereof further comprises a light chain constant region of the human κ or λ chain or the variant thereof, preferably a human κ light chain constant region, wherein more preferably, the amino acid sequence of the human κ light chain constant region is set forth in SEQ ID NO: 19, or has at least 90%, at least 95%, at least 96%, at least 97%, at least 98% or at least 99% sequence identity to SEQ ID NO: 19.

6. The antibody-drug conjugate or the pharmaceutically acceptable salt or solvate thereof according to any one of claims 1-5, wherein the linker L is represented by general formula (L): 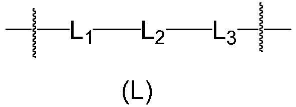 wherein
L₁ is selected from 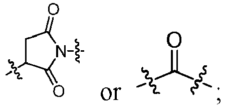
L₂ is 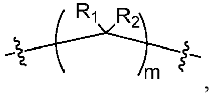 wherein R₁ and R₂ are each independently selected from H, alkyl, haloalkyl, or halogen, and m is 1-8, preferably 2-5; L₂ is preferably 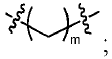 or
L₂ is 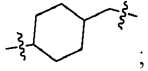
L₃ is selected from' 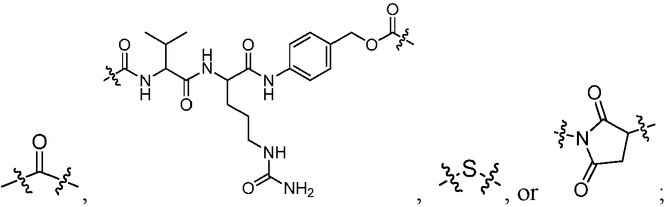 preferably, the linker L is selected from the following structures: 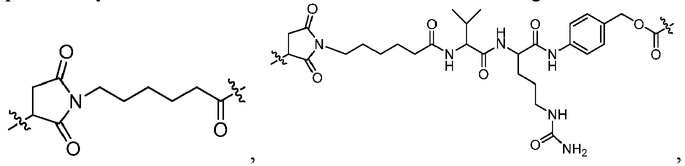 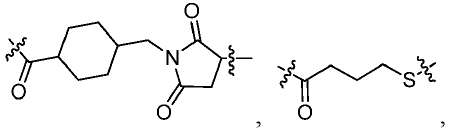 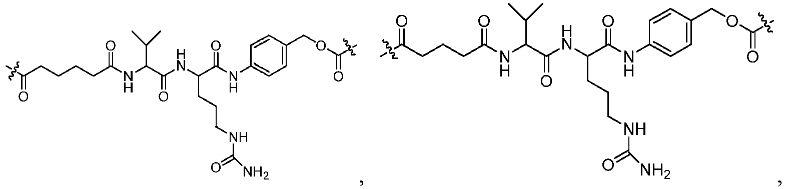 and

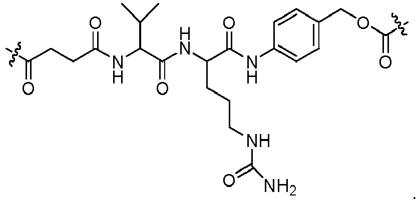

7. The antibody-drug conjugate or the pharmaceutically acceptable salt or solvate thereof according to any one of claims 1-5, wherein the cytotoxin molecule D is selected from a chemotherapeutic agent, a DNA alkylating agent, a tubulin inhibitor, a topoisomerase inhibitor, an antibiotic, or a cytotoxic agent of a radioisotope;
preferably, the cytotoxin molecule D is selected from the following structures:

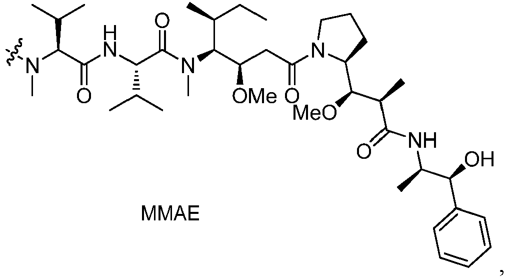

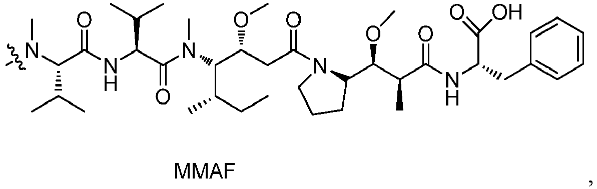

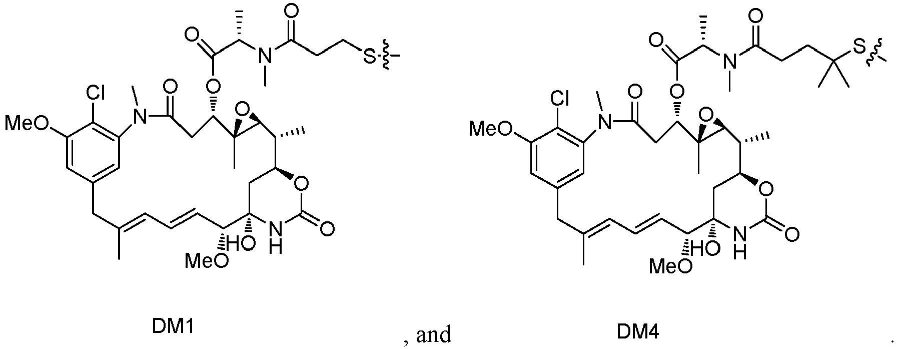

8. The antibody-drug conjugate or the pharmaceutically acceptable salt or solvate thereof according to any one of claims 1-7, wherein the antibody-drug conjugate or the pharmaceutically acceptable salt or solvate thereof is an antibody-drug conjugate represented by general formula (II) or a pharmaceutically acceptable salt or solvate thereof: 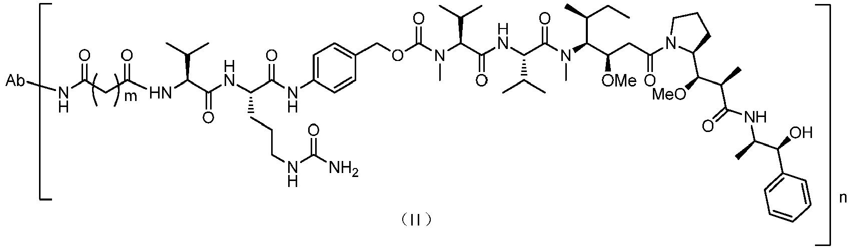
wherein m is 1-8, preferably 2-4; 0 < n ≤ 10, preferably 0.8 ≤ n ≤ 3, and Ab is as defined in any one of claims 1-5; or
the antibody-drug conjugate or the pharmaceutically acceptable salt or solvate thereof is an antibody-drug conjugate represented by general formula (III) or a pharmaceutically acceptable salt or solvate thereof: 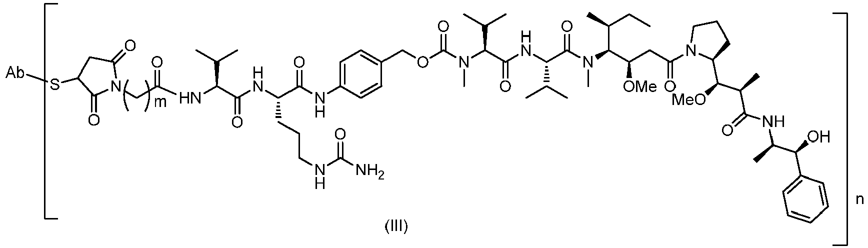
wherein m is 1-8, preferably 3-5, 0 < n ≤ 8, and Ab is as defined in any one of claims 1-5; or
the antibody-drug conjugate or the pharmaceutically acceptable salt or solvate thereof is selected from the following structures: 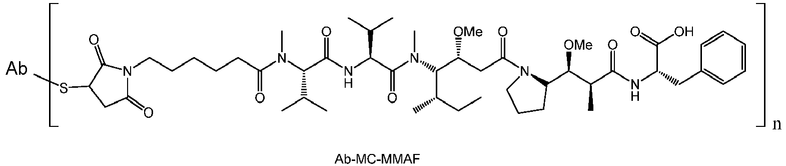
wherein 0 < n ≤ 8, and Ab is as defined in any one of claims 1-5; 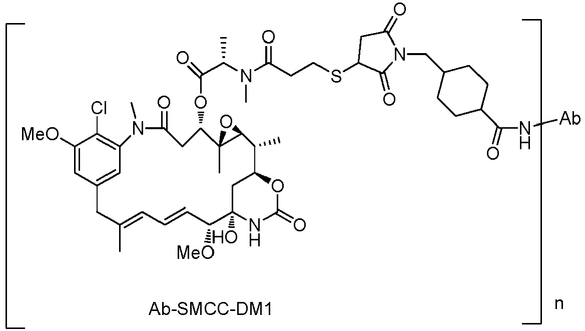
wherein 0 < n ≤ 10, and Ab is as defined in any one of claims 1-5; and 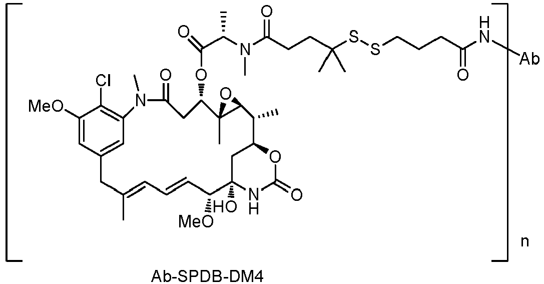
wherein 0 < n ≤ 10, and Ab is as defined in any one of claims 1-5; preferably, the antibody-drug conjugate is: 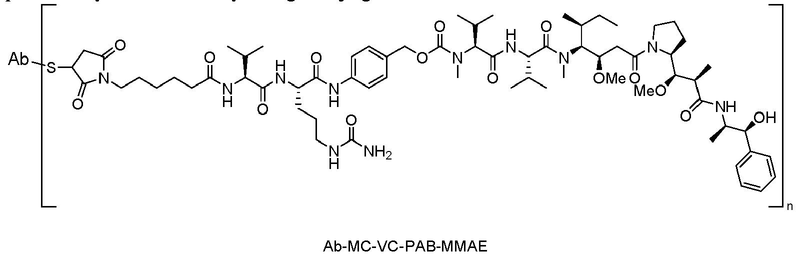
wherein 0 < n ≤ 8, and Ab is as defined in any one of claims 1-5; 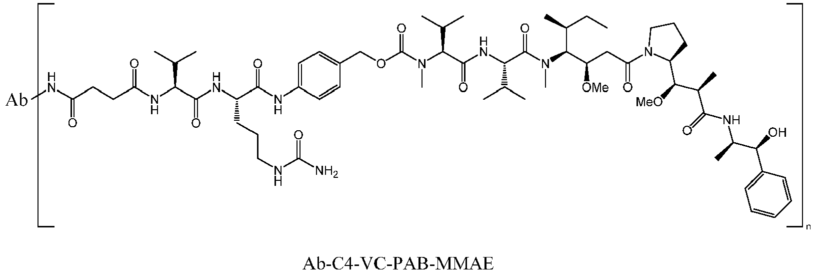
wherein 0 ≤ n ≤ 10, preferably 0.8 ≤ n ≤ 3, and Ab is as defined in any one of claims 1-5; 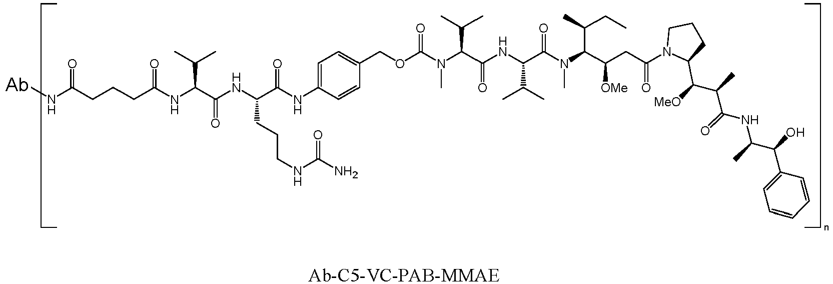
wherein 0 < n ≤ 10, preferably 0.8 ≤ n ≤ 3, and Ab is as defined in any one of claims 1-5; or 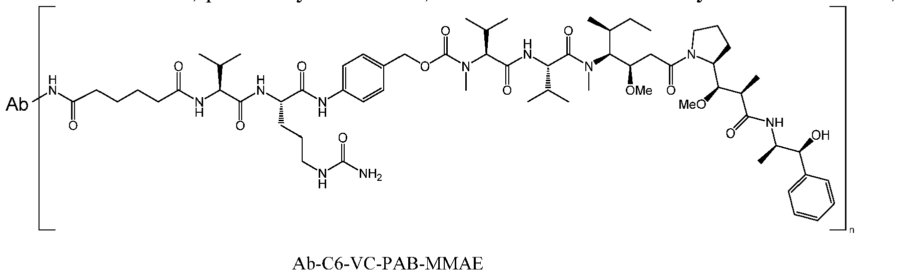
wherein 0 < n ≤ 10, preferably 0.8 ≤ n ≤ 3, and Ab is as defined in any one of claims 1-5; more preferably, the antibody-drug conjugate is:
(1) F314-MC-VC-PAB-MMAE with a structure shown in the following formula: 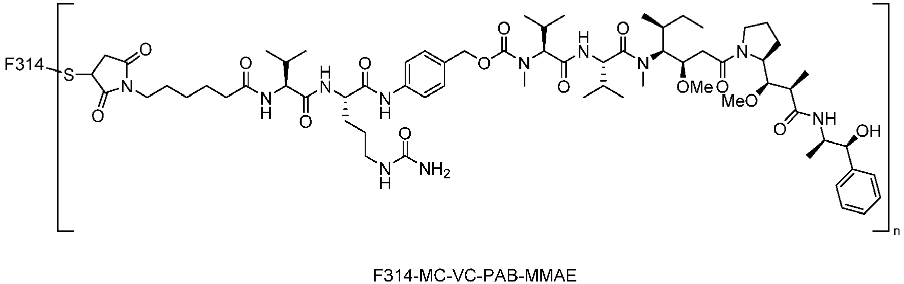 wherein n is 3.8-4.2, preferably 3.9-4.1, more preferably 4.0;
(2) F314-MC-MMAF with a structure shown in the following formula: 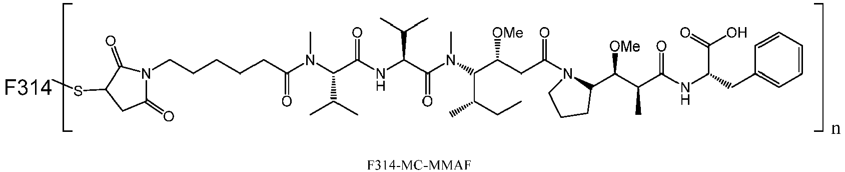 wherein n is 1.3-1.7, preferably 1.4-1.6, preferably 1.5;
(3) F314-SMCC-DM1 with a structure shown in the following formula: 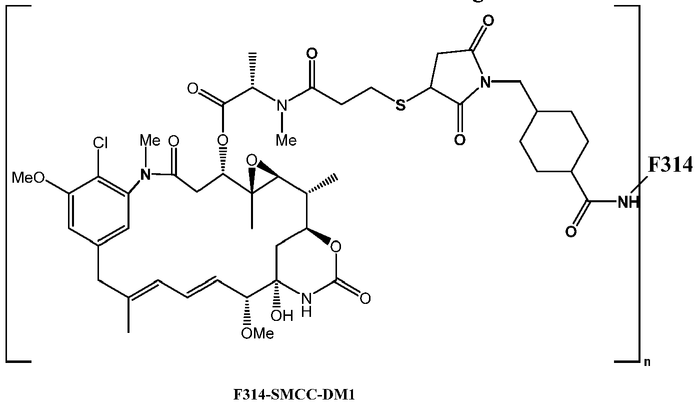 wherein n is 4.8-5.2, preferably 4.9-5.1, more preferably 5.0;
(4) F314-SPDB-DM4 with a structure shown in the following formula: 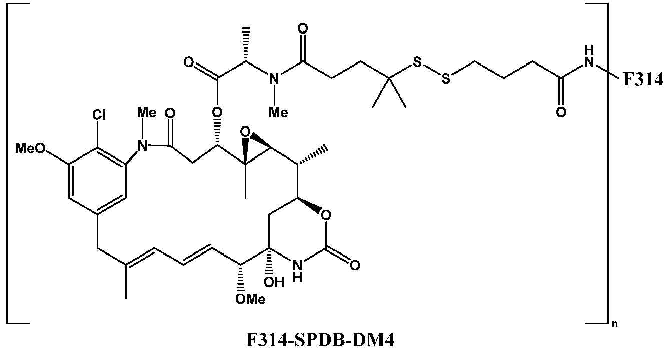 wherein n is 3.6-4.0, preferably 3.7-3.9, more preferably 3.8;
(5) F314-C4-VC-PAB-MMAE with a structure shown in the following formula: 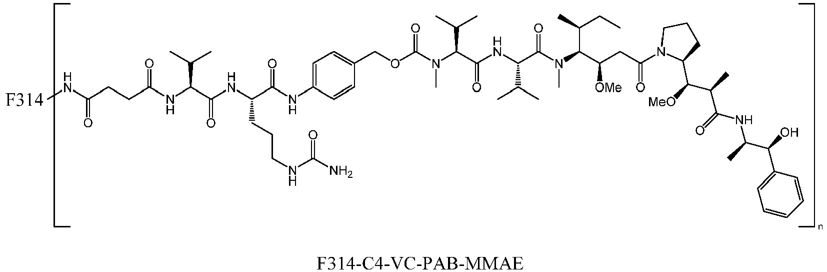 wherein n is 1.9-2.3, preferably 2.0-2.2, more preferably 2.1;
(6) F314-C5-VC-PAB-MMAE with a structure shown in the following formula: 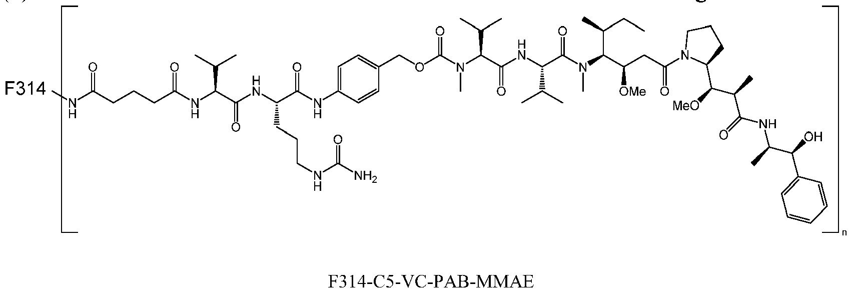 wherein n is 0.5-3.5, preferably 0.8-3.0, more preferably 0.8, 1.8, 2.3 or 3.0; or
(7) F314-C6-VC-PAB-MMAE with a structure shown in the following formula: 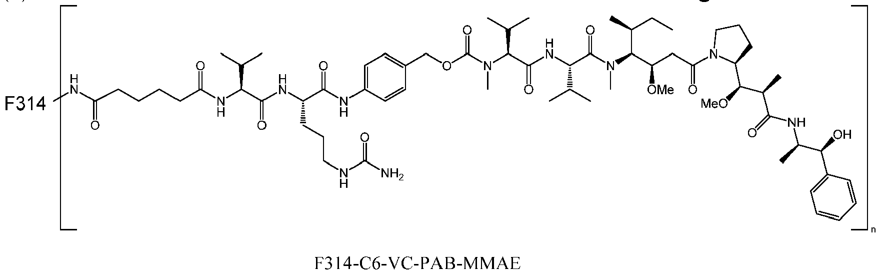
wherein n is 1.9-2.3, preferably 2.0-2.2, more preferably 2.1;
wherein, in each formula, F314 represents a humanized monoclonal antibody F314, which has a heavy chain variable region with the amino acid sequence set forth in SEQ ID NO: 14, a light chain variable region with the amino acid sequence set forth in SEQ ID NO: 17, a heavy chain constant region with the amino acid sequence set forth in SEQ ID NO: 18, and a light chain constant region with the amino acid sequence set forth in SEQ ID NO: 19.

9. A pharmaceutical composition, comprising the antibody-drug conjugate or the pharmaceutically acceptable salt or solvate thereof according to claims 1-8, and a pharmaceutically acceptable excipient, diluent or carrier.

10. Use of the antibody-drug conjugate or the pharmaceutically acceptable salt or solvate thereof according to claims 1-8 or the pharmaceutical composition according to claim 9 in the preparation of a medicament for treating a CD39-mediated disease or disorder, wherein the disease or disorder is preferably a cancer, further preferably lymphoma, multiple myeloma, thyroid cancer, colorectal cancer, gastric cancer, renal cancer, prostate cancer, testicular cancer, breast cancer, ovarian cancer, or melanoma.

11. A method for treating and preventing a CD39-mediated disease or disorder, comprising administering to a patient in need thereof a therapeutically effective amount of the antibody-drug conjugate or the pharmaceutically acceptable salt or solvate thereof according to claims 1-8, or comprising the pharmaceutical composition according to claim 9; wherein the disease or disorder is preferably a cancer, further preferably lymphoma, multiple myeloma, thyroid cancer, colorectal cancer, gastric cancer, renal cancer, prostate cancer, testicular cancer, breast cancer, ovarian cancer, or melanoma.
